# EUROPEAN PATENT APPLICATION

(11) **EP 3 546 478 A1**
(43) Date of publication of application: **02.10.2019**
(21) Application number: 18000305.5
(22) Date of filing: 30.03.2018
(51) Int. Cl.: C07K 14/49, A61K 38/18

(54) **NEW PEPTIDE DERIVATIVES OF PLATELET DERIVED GROWTH FACTOR (PDGF), A METHOD FOR THEIR PREPARATION, PHARMACEUTICAL COMPOSITIONS CONTAINING THEM AND APPLICATION OF THESE COMPOSITIONS**

(30) Priority: 26.03.2018 PL 42503818
(71) Applicant: Uniwersytet Gdanski, 80-309 Gdansk (PL); Gdanski Uniwersytet Medyczny, 80-210 Gdansk (PL); Politechnika Gdanska, 80-233 Gdansk (PL); Instytut Biologii Doswiadczalnej Im. M. Nenckiego Polskiej Akademii Nauk, 02-093 Warszawa (PL); MEDVentures sp. z. o. o., 60-141 Poznan (PL); Pro Science Polska Sp. Z O.O., 80-302 Gdarnsk (PL)
(72) Inventor: Rodziewicz-Motowidlo, Sylwia, 83-010 Rotmanka (PL); Pikula, Michal, 80-170 Gdansk (PL); Deptula, Milena, 80-180 Gdansk (PL); Karpowicz, Przemyslaw, 11-200 Bartoszyce (PL); Sas, Piotr, 81-577 Gdynia (PL); Wardowska, Anna, 80-297 Banino (PL); Sawicka, Justyna, 80-174 Gdansk (PL); Dzierzynska, Maria, 81-824 Sopot (PL); Kasprzykowski, Franciszek, 83-000 Pruszcz Gdanski (PL); Sosnowski, Pawel, 19-200 Ruda (PL); Mieczkowska, Alina, 80-126 Gdansk (PL); Filipowicz, Natalia, 80-262 Gdansk (PL); Madanecki, Piotr, 80-180 Kowale (PL); Piotrowski, Arkadiusz, 83-031 Rozyny (PL); Czypryn, Artur, 02-747 Warszawa (PL); Mucha, Piotr, 83-110 Tczew (PL); Skowron, Piotr, 80-303 Gdansk (PL); Janus, Lukasz, 60-694 Poznan (PL); Sachadyn, Pawel, 81-646 Gdynia (PL)
(74) Representative: Matyka, Malgorzata

(57) **Abstract**

The present invention relates to novel peptide derivatives of platelet derived growth factor (PDGF) and a method for their preparation.

In addition, the invention relates to a pharmaceutical composition containing the new compounds and the use of these compounds as a treatment for skin problems that are difficult to heal and skin lesions of various aetiologies, including wounds resulting from venous insufficiency, tissue ischemia, atherosclerosis, diabetes, neurological and inflammatory diseases, surgical wounds, wounds after chemo-and/or radiotherapy treatments and other pathological conditions.

## Description

### AREA OF TECHNOLOGY

The subject of the invention is new peptide derivatives of platelet derived growth factor and a method of preparing them. Furthermore, the subject of the invention is a pharmaceutical composition comprising the new compounds and the use of these compounds as a therapeutic agent for the regeneration and/or treatment of wounds caused by mechanical, chemical, thermal and/or radiative injuries, surgical operations and/or other pathological conditions.

### STATE OF TECHNOLOGY

There is currently a significant need for the development of new methods of treatment of injuries requiring surgical interventions, such as thermal, chemical or radiation burns of large area or chronic wounds resulting from civilization diseases such as diabetes or ischemia. Stimulation of regeneration and non-scar wound healing may be such a method.

Wound healing is a dynamic process consisting of several phases, among which three phases are distinguished as the main ones: inflammation, proliferation and remodeling. There are many factors participating in its control. Growth factors play an important role in the process of wound healing by regulating the proliferation and differentiation of cells. A special role is played by platelet-derived growth factor (PDGF), which is produced mainly by thrombocytes. It was discovered in the 1970's and participates in almost all phases of wound healing. The family of PDGF factors encompasses five homodimeric and heterodimeric proteins: PDGF-AB, PDGF-AA, PDGF-BB, PDGF-CC and PDGF-DD. The main role of these proteins is the direct binding of transmembrane receptors of protein kinase (receptors PDGF-α and PDGF-β) and their activation. After an injury, PDGF is released in large amounts by degranulating platelets and is present in the discharge from the wound, especially in the early stagesafter the injury. Its source may also be keratinocytes, fibroblasts, endothelial cells and macrophages.

PDGF acts as a mitogen for a variety of cells such as fibroblasts, keratinocytes or endothelial cells. It causes the chemotaxis of neutrophils, macrophages, fibroblasts and smooth muscles cells to the wounded area, which helps with the commencement of the inflammatory phase of wound healing. Moreover, it acts chemotactically on the mesenchymal stem cells of the bone marrow, which accumulate mainly in the wound and may differentiate into fibroblasts. During the epithelization, it stimulates the secretion of insulin-like growth factor (IGF), which increases the mobility of keratinocytes and trombospodine1. PDGF also increases the proliferation of fibroblasts and, as a consequence, the production of extracellular matrix as well as the induction of the change of the phenotype of fibroblasts into myofibroblasts, which facilitates wound contraction.

In addition, PDGF stimulates the production of components of the extracellular matrix such as fibronectin, collagen, proteoglycan and hyaluronic acid. It also increases the production and secretion of collagenases by fibroblasts, which suggests its role in the remodeling phase. A lowered concentration of PDGF, due to its sensitivity to the proteolytic environment typical of chronic wounds, is observed in patients with diabetic foot ulcer.

Recombinant human PDGF-BB in the form of a hydrogel (Bakaplermin, Regranex, Johnson&Johnson) is the only commercially available growth factor approved by the FDA. It was approved in 1997 for topical use in the treatment of ulceration of the lower limbs in diabetic wounds. However, this product is not widely used due to high costs of production and limited efficacy. Moreover, it has been observed that the application of three or more packages of Regranex is connected with a higher risk of the development of a variety of skin cancers. It has led to a warning issued by the FDA informing patients about this connection.

Peptides have been used for many years in the process of wound healing. Due to their mechanism of action and their origin, several classes can be distinguished: (i) peptides with antimicrobial/regenerative activity; (ii) compounds based on the sequence of collagen and elastin; (iii) derivatives of growth factors/hormones and proteins and peptides involved in the immune and hematopoietic processes; and (iv) compounds of animal origin. The following are examples of peptides from each class:
Examples of peptides with antibacterial and regenerative properties:Examples of peptides with antibacterial and regenerative properties:
   AG30 (MLSLIFLHRLKSMRKRLDRKLRLWHRKNYP); AH90 (ATAWDFGPHGLLPIRPIRIRPLCG); CW49 (APFRMGICTTN); Cys-KR12 (KRIVKRIKKWLR); Esculentin-1a (1-21) (GIFSKLAGKKIKNLLISGLKG); Histatin-2 (RKFHEKHHSHREFPFYGDYGSNYLYDN); IDR-1018 (VRLIVAVRIWRR); SHAP1 (APKAMKLLKKLLKLQKKGI); Temporin A (FLPLIGRVLSGIL); and Temporin B (LLPIVGNLLKSLL)
Compounds based on the sequence of collagen and elastin:
   GHK, BioGHK (Biotin-GHK); collagen; chitosan; Ac-PGP (N-acetyl-PGP); Comb1 (DINECEIGAPAGEETEVTVEGLEPG); and E1 (GETGPAGPAGPIGPVGARGPAGPQGPRGDKGETGEQ);
Compounds of animal origin:
   Tiger17 (c [WCKPKPKPRCH-NH2]); andTylotoin (KCVRQNNKRVCK);
Derivatives of growth factors / hormones and proteins and peptides involved in the processes of the immune and hematopoietic system:
   TP-508 (AGYKPDEGKRGDACEGDSGGPFV); TSN1 (NFQGVQNRFVFGTP), TNS2 (MENAELDVPIQSVFTR); UN2 (TATSEYQTFFNPR); WKYMVm (WKYMV-D-M); a derivative of angiopoetin-1 (QHREDGS); Pep2-YAC (YAC) (EGF derivative); RGD; and various derivatives of growth factors.

The stability of a pharmaceutical formulation is defined as the period from its manufacture until it does not meet the criteria specified in the Pharmacopoeia (or other guidelines) or when the predetermined concentration of the active substance decreases by more than 10%. Moreover, the formulation must remain within its specifications with regard to physical form, taste, bioavailability or toxicity. The stability tests are performed at defined time points (stability profile) and under specific conditions of temperature, humidity, pH, etc., to prove that the pharmaceutical formulation remains effective and safe for patients. Collected data are the basis for determining the durability of the product and its expiration date.

Stability tests of the active product ingredient (API) might be performed in the form of a solid, e.g. with fillers (tablets), an emulsion (creams) or dissolved in water or a buffer. Very valuable information on API stability is provided by plasma and/or whole blood tests. These kinds of tests allow assessment of the susceptibility of the compound to enzymatic proteolysis and determination of the suitable drug dosage to achieve the desired pharmacological effect. Compounds characterized by a short half-life time (t_{1/2}) generally require further structural modifications (not true for prodrugs) to improvetheir stability parameters. However, false short half-life time results are frequently due to binding of the API to blood plasma proteins, e.g. albumins nor degradation.

Albumins are essential proteins, with a molecular mass around 66-69 kDa, found in human plasma. They account for about 50%-60% of plasma proteins. Their basic physiological function is binding of water so that the colloidosmotic (oncotic) plasma pressure and its normal volume are maintained. They also serve as the main carriers of free fatty acids, hormones, vitamins, heavy metals and certain drugs (e.g. acetylsalicylic acid). Most drugs bind reversibly to these proteins via hydrogen bonds, hydrophobic interactions or van der Waals forces, creating a so-called depot (storage), from which the drug particles are gradually released, replacing those that have already been eliminated. Currently, attention is increasingly focused on albumin as exogenous drug carriers, as they are not toxic or immunogenic, and they also improve the pharmacokinetic profile (t_{1/2} of albumin is about 15-29 days) and the stability of drugs which are rapidly degraded in plasma. Albumins are robust proteins because of their extraordinary stability over a wide pH range (4-9), their temperature stability (they can be heated at 60°C for up to 10 h), and their resistance to most denaturing agents and solvents at moderate concentrations. All this factors together makes albumins very attractive in the manufacture of drugs.

### DETAILED DESCRIPTION OF THE INVENTION

The present invention relates to amino acid sequences of synthetic peptides whose amino acid sequences are fragments of growth factors that interact with the respective receptor and accelerate wound healing. Additionally, the invention relates to their use in wound healing in mammals.

Synthetic peptide derivatives of PDGF protein (hereinafter referred to as PDGF1, Ac-PDGF1, PDGF2, PDGF2-HTT, PDGF2-NE and PDGF3), designed on the basis of the PDGF protein spatial structure (in the PDGF-BB complex), are good candidates for use as active compounds instimulating wound healing They can be subjected to chemical modifications in order to increase their activity or reduce the side effects of their use.

In our studies, *anin vitro* cell model and an animal model were used. The cellular model employed mainly human keratinocytes and fibroblasts - cells crucial for wound healing and skin regeneration. The presence of these cells, and especially their proliferation (division), is essential for the proper wound healing process. In the animal model, analysis of wound healing on the mouse back was performed. This model is widely approved in the study of new compounds - potential drugs that stimulate wound healing in humans. Due to the similar physiology and biochemistry of wound healing in mice and humans, the animal model can be the basis for assessing the effectiveness of new compounds. In addition, animal models give a high reproducibility of results and allow control of conditions and standardization of experiments, compared to human studies.

The subject of the following invention is a new compound with the general formula:

**xₙ[A-/-B]yₙ**

where:
Xₙ - represents a functional group/protecting group at the *N*-terminus of the compound, for example, for n=1 H-, for n=2 Ac-, for n=3 -PEG2-;
Yₙ- represents a functional group/protecting group at the C-terminus of the compound, for example, for n=1 - NH₂, for n=2 - COOH;
A - represents a moiety containing sequence of **R¹K²I³E⁴I⁵V⁶R⁷K⁸K⁹P¹⁰I¹¹F¹² ,** or its fragments;
B - represents a moiety containing sequence of **R¹L²I³D⁴R⁵T⁶N⁷A⁸N⁹F¹⁰L¹¹** , or its fragments; -/- means the variation of the order and ways of combining A and B, A with A, B with A in any order and multiplicity; the methods of combining the motifs include: combinations of polyethylene glycol motif(PEG), polypeptide, engaging side chains of amino acids of any length and sequence, as well as motif A and/or B in a cyclic form.

The subject of the invention is also amethod for the preparation of a new compound as defined above, comprising the following steps:
a) swelling of TentaGel R RAM amide resin;
b) deprotecting the Fmoc protecting group with 20% piperidine in DMF;
c) resin washing with DMF (4x);
d) performing a chloranil test;
e) coupling reaction of Fmoc-protected amino acid assisted with microwave irradiation and the use of 0.5 M solution of DIC in DMF and 1M Oxyma Pure;
f) resin washing with DMF (4x);
g) performing a chloranil test;
h) repeating steps b) to g) until the peptide of claim 1 is obtained;
i) cleaving the peptide from the support while removing the protecting groups from the side-chains with a mixture of: TFA:TIPSI:H₂O (92:4:4, v/v/v);
j) draining, precipitating with cooled ether, washing the precipitate three times with Et₂O, dissolving and freeze-drying;
k) analysing and purifying with HPLC;
l) confirming the product identity with mass spectrometry

The present invention also relates to:
A. a pharmaceutical composition which comprises a novel compound as defined above and at least one pharmaceutically acceptable carrier and / or diluent.
B. A pharmaceutical composition that can be applied locally or systemically such as: topically, intravenously, orally, parenterally and / or in the form of implants, as well as rectal use.
C. A pharmaceutical composition where local or system applications can be in solid and / or liquid form.
D. A composition that is used to stimulate wound healing.
E. The present invention relates to the *in vitro* use of a new compound as defined above for the detection of stimulation of healing of wounds caused by mechanical, chemical, thermal or radiative injuries, surgical operations or pathological conditions.
F. Application where the stimulation of wound healing occurs through accelerated epithelialization.
G. Application where the healing of wounds includes skin wounds, epidermis, hair follicles, hair, sweat glands.
H. The new compounds defined above for use as a means to stimulate the healing of skin wounds, epidermis, hair follicles, hair, sweat glands.

The terms used in the present description and the claims have the following meanings:
The term "wound" includes any kind of damage to living tissue caused by impact or pressure and other mechanical factors, burns, frostbite, radiation, chemical agents, ischemia and other biological agents. In particular, the term "wound" includes damage to the skin, but also to other tissues and organs.

The term "wound healing" refers to a post-injury repair process that involves cleansing the wound area from foreign bodies and dead tissues and cell proliferation leading to the recreationof tissue continuity either to form a scar or not.

The term "regeneration" means the process of reconstructing lost tissues, structures and appendages occurring spontaneously or under the influence of pharmacological stimulation.

The term "chronic wound" or "non-healing wound" refers to wounds that show a deficit of healing relative to normal healing; typically wounds that do not heal within 3 months are referred to as chronic. Chronic wounds or non-healing wounds are often associated with ischemia or ulceration in diabetic patients,but are not limited to this group.

In the case where the compound is administered in the early stages of wound healing, it should be understood that the term "early phase" includes an immune response immediately after wounding.

### PDGF - Platelet-derived Growth Factor

Peptidomimetic - compound with a structure mimicking a peptide containing non-proteinaceous amino acids.
DMF - *N,N*-Dimethylformamide
MeOH - methanol
DIPEA - *N,N*-Diisopropylethylamine
Fmoc - 9H-fluoren-9-yl-methyloxycarbonyl group
Boc - *tert*-butyloxycarbonyl group
DIC - *N,N'*-diisopropylcarbodiimide
Et₂O - diethyl ether
Pbf - 2,2,4,6,7-pentamethyldihydrobenzofuran-5-sulfonyl group
O*t*Bu - tert-butyl ester group
ACN - acetonitrile
TIPSI - triisopropylsilane
TFA - trifluoroacetic acid
Rₜ- Retention time
Ac - acetyl group
Ac₂O - acetic anhydride
*t*-Bu - *tert-butyl* group
KI - potassium iodide
HATU - 1-[Bis(dimethylamino)methylene]-1H-1,2,3-triazolo[4,5-b]pyridinium 3-oxid hexafluorophosphate
HOAt - 1-Hydroxy-7-azabenzotriazole
Oxyma pure - ethyl(hydroxyimino)cyanoacetate
DCM - dichloromethane
Mtt - 4-Methyltrityl group
MS - mass spectrometry
LDH - lactate dehydrogenase
XTT- 2,3-Bis-(2-Methoxy-4-Nitro-5-Sulfophenyl)-2*H*-Tetrazolium-5-Carboxanilide
API - active pharmaceutical ingredient
HaCaT - immortalized human keratinocyte cell line
FBS- Fetal Bovine Serum
PBS- Phospahte-Buffered Saline
PBMC- peripheral blood mononuclear cell
fMLP-*N*-Formylmethionyl-leucyl-phenylalanine
P/S- penicillin/streptomycin
ASA- acetylsalicylic acid
LPS- Lipopolysaccharides
PHA- Phytohaemagglutinin
BAT- Basophil Activation Test
ASC -Adipose Derived Stem Cells
P407 - poloxamer-407 (Poly(ethylene glycol)-*block*-poly(propylene glycol)-*block*-poly(ethylene glycol))

### Figure description:

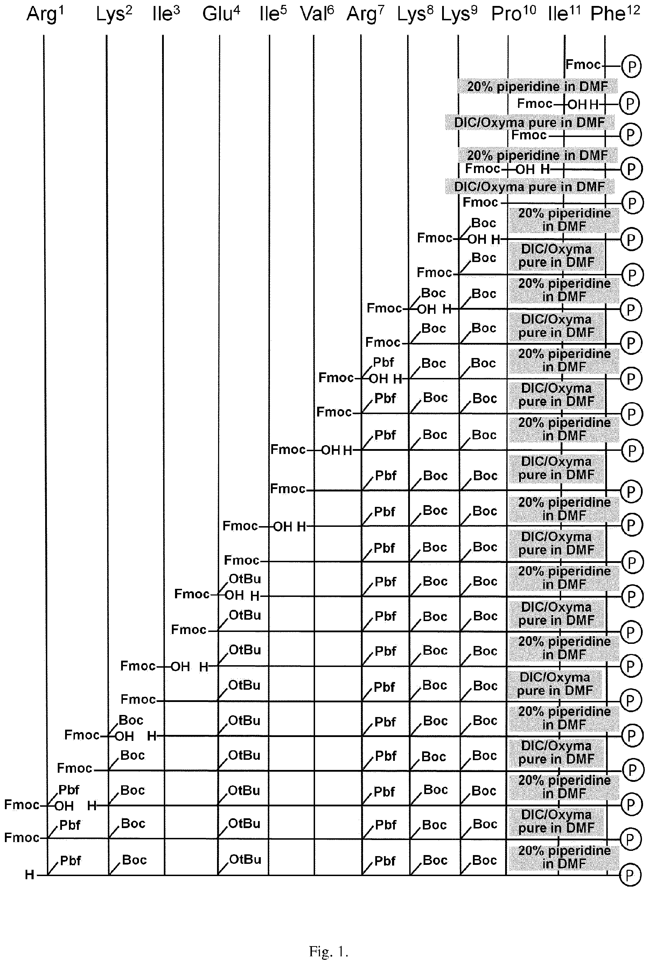

**Fig.1** -presents the synthesis scheme of the PDGF1 compound

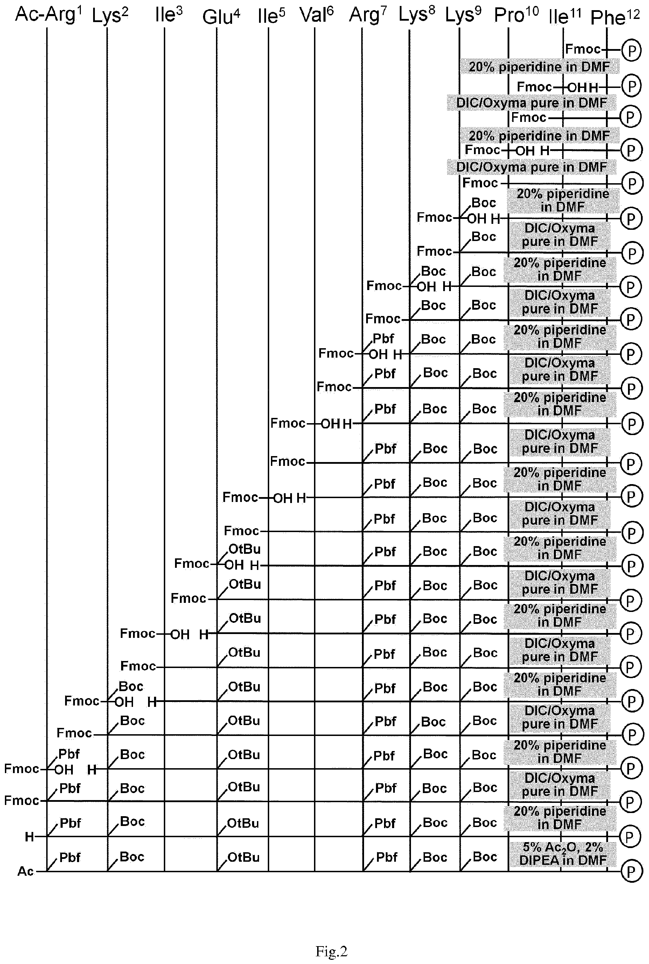

**Fig.2** - presents the synthesis scheme of the Ac-PDGF1 compound

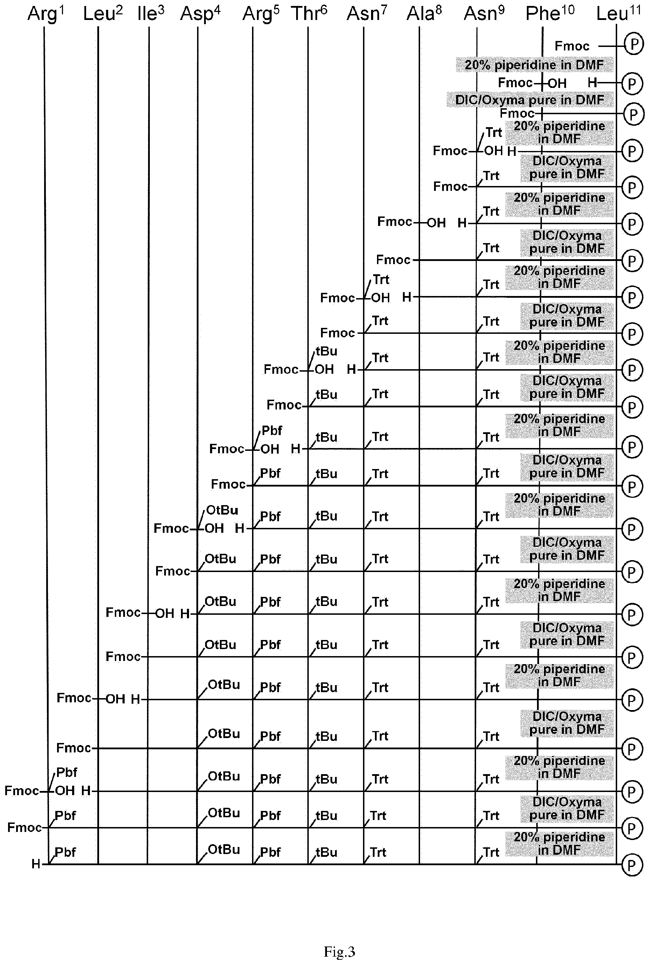

**Fig.3** - presents the synthesis scheme of the PDGF2 compound

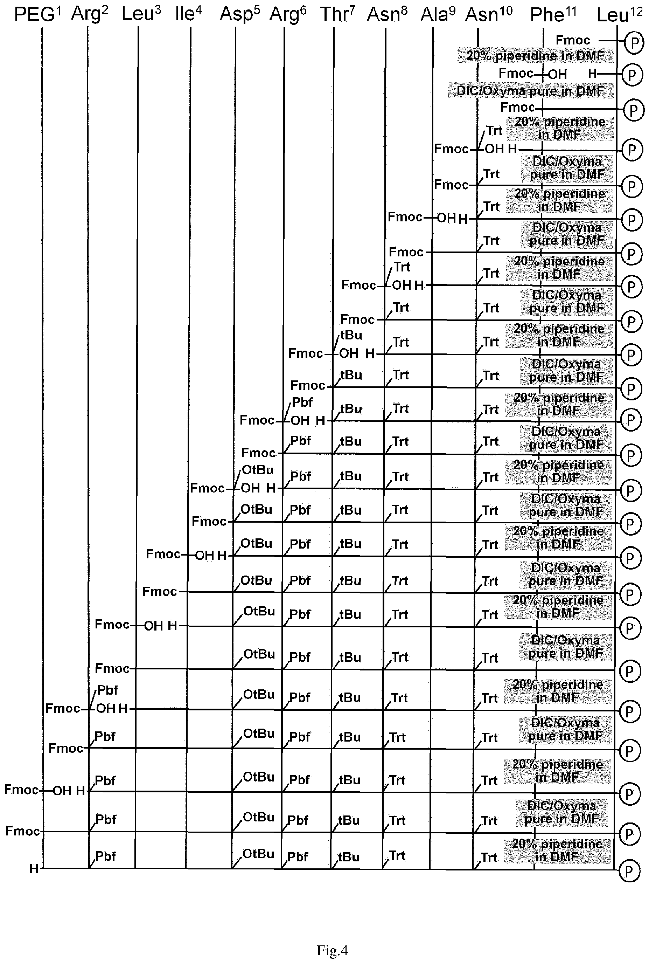

**Fig.4** - presents the synthesis scheme of the PDGF2-HTT compound
**Fig.5** - presents the synthesis scheme of the PDGF2-NE compound

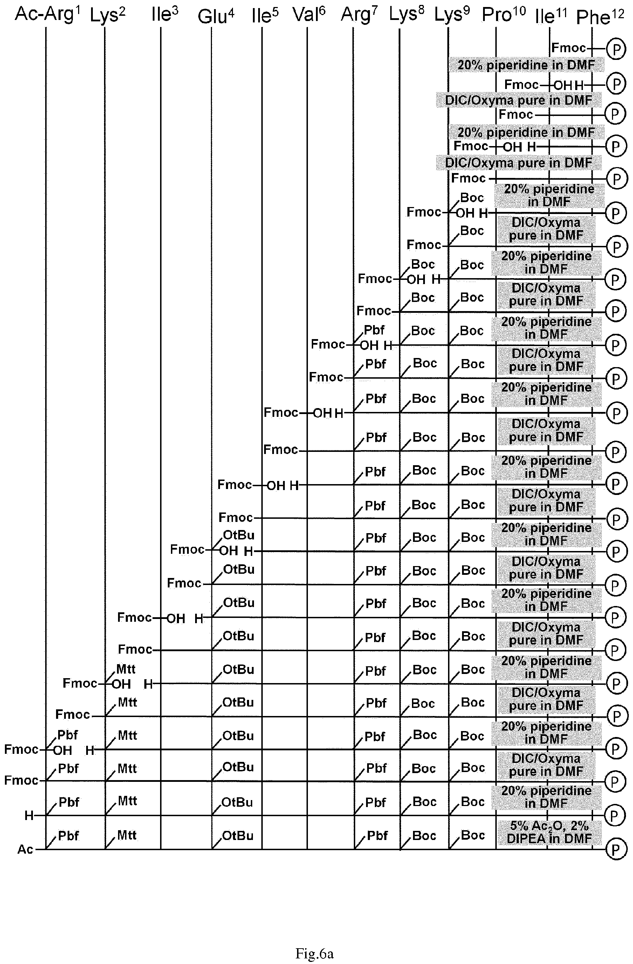

**Fig.6a****-** presents the synthesis scheme of the PDGF3 compound - fragment 1

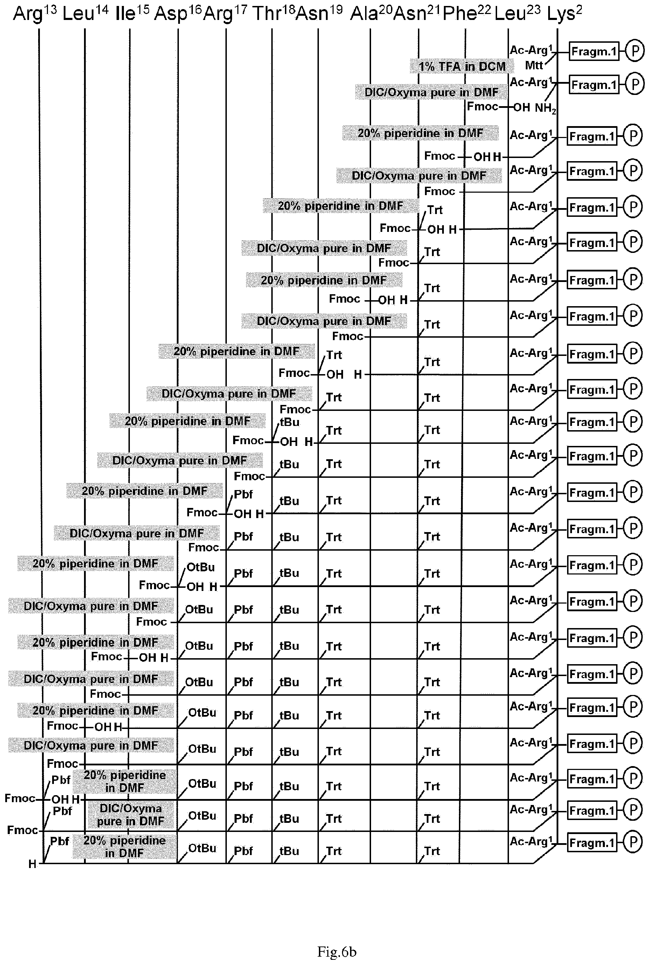

**Fig.6b** - presents the synthesis scheme of the PDGF3 compound - fragment 2

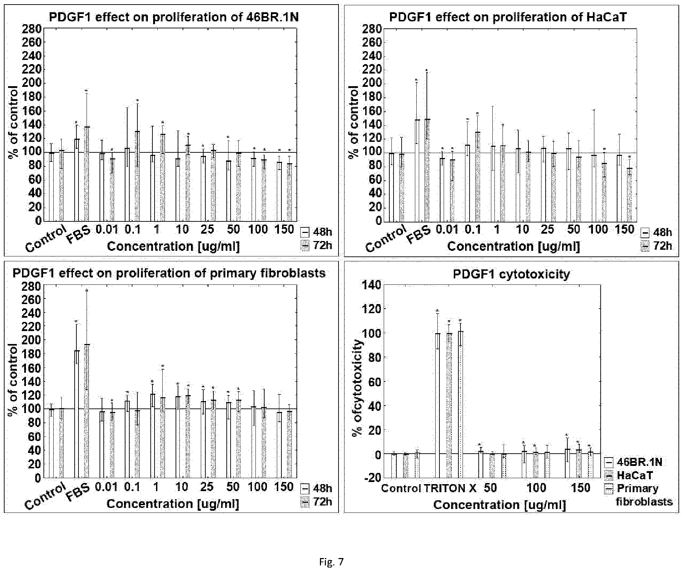

**Fig. 7** - presents graphs showing the effect of PDGF1 on proliferation (XTT test) of 46BR.1N fibroblasts, HaCaT keratinocytes and primary fibroblasts, and its cytotoxicity to these cells (LDH test). The graphs show a summary of results from 4 cycles of experiments (4 replicates in each, n = 16). * - statistically significant differences compared to the control (cells cultured in medium without serum), U-Mann Whitney's test (p <0.05, n = 16); FBS - positive control in the XTT test (cells grown in medium with 10% serum), TRITON X - positive control in the LDH test (cells incubated in medium with the addition of 1% triton X, maximum cytotoxicity).

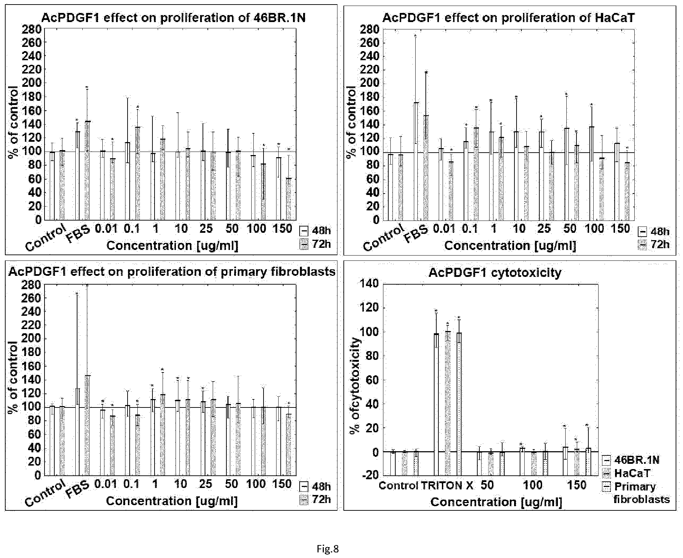

**Fig. 8** - presents graphs showing the effect of AcPDGF1 effect on proliferation (XTT test) of 46BR.1N fibroblasts, HaCaT keratinocytes and primary fibroblasts, and its cytotoxicity to these cells (LDH test). The graphs show a summary of results from 4 cycles of experiments (4 replicates in each, n = 16). * - statistically significant differences compared to the control (cells cultured in medium without serum), U-Mann Whitney's test (p <0.05, n = 16); FBS - positive control in the XTT test (cells grown in medium with 10% serum), TRITON X - positive control in the LDH test (cells incubated in medium with the addition of 1% triton X, maximum cytotoxicity).

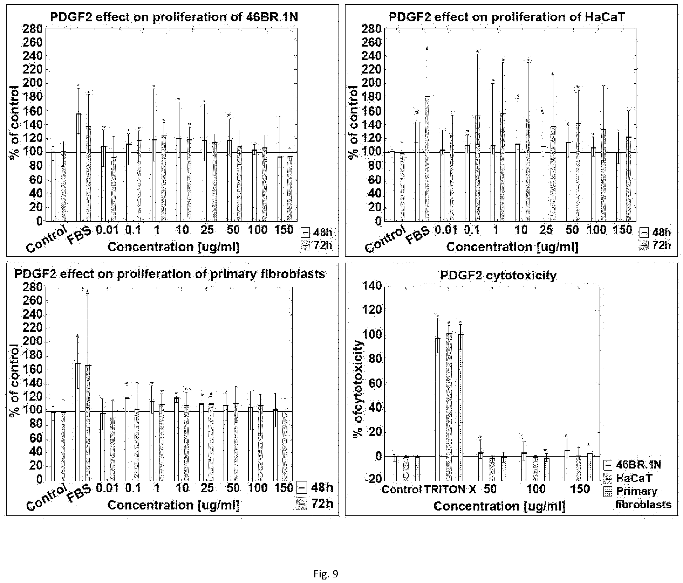

**Fig. 9** **-** presents graphs showing the effect of PDGF2 on proliferation (XTT test) of 46BR.1N fibroblasts, HaCaT keratinocytes and primary fibroblasts, and its cytotoxicity to these cells (LDH test). The graphs show a summary of results from 4 cycles of experiments (4 replicates in each, n = 16). * - statistically significant differences compared to the control (cells cultured in medium without serum), U-Mann Whitney's test (p <0.05, n = 16); FBS - positive control in the XTT test (cells grown in medium with 10% serum), TRITON X - positive control in the LDH test (cells incubated in medium with the addition of 1% triton X, maximum cytotoxicity).

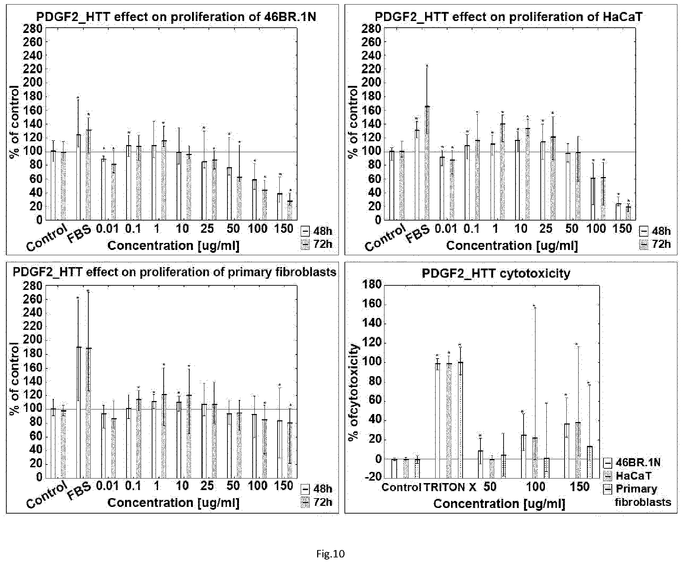

**Fig. 10****-** presents graphs showing the effect of PDGF2_HTT on proliferation (XTT test) of 46BR.1N fibroblasts, HaCaT keratinocytes and primary fibroblasts, and its cytotoxicity to these cells (LDH test). The graphs show a summary of results from 4 cycles of experiments (4 replicates in each, n = 16). * - statistically significant differences compared to the control (cells cultured in medium without serum), U-Mann Whitney's test (p <0.05, n = 16); FBS - positive control in the XTT test (cells grown in medium with 10% serum), TRITON X - positive control in the LDH test (cells incubated in medium with the addition of 1% triton X, maximum cytotoxicity).

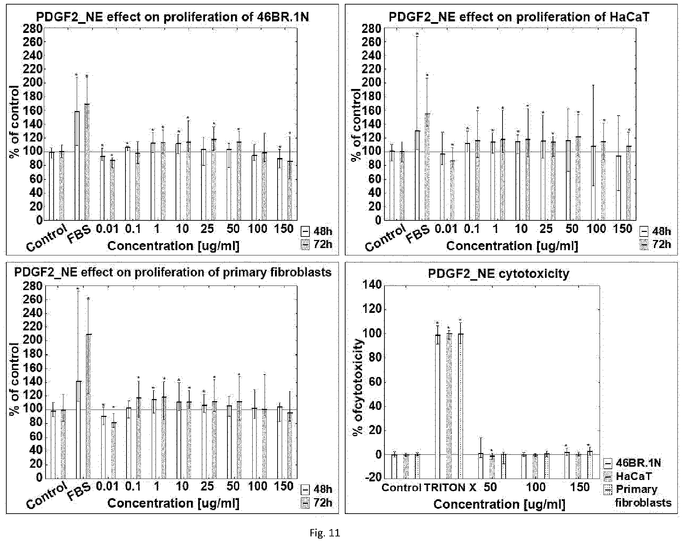

**Fig. 11** **-** presents graphs showing the effect of PDGF2_NE on proliferation (XTT test) of 46BR.1N fibroblasts, HaCaT keratinocytes and primary fibroblasts, and its cytotoxicity to these cells (LDH test). The graphs show a summary of results from 4 cycles of experiments (4 replicates in each, n = 16). * - statistically significant differences compared to the control (cells cultured in medium without serum), U-Mann Whitney's test (p <0.05, n = 16); FBS - positive control in the XTT test (cells grown in medium with 10% serum), TRITON X - positive control in the LDH test (cells incubated in medium with the addition of 1% triton X, maximum cytotoxicity).

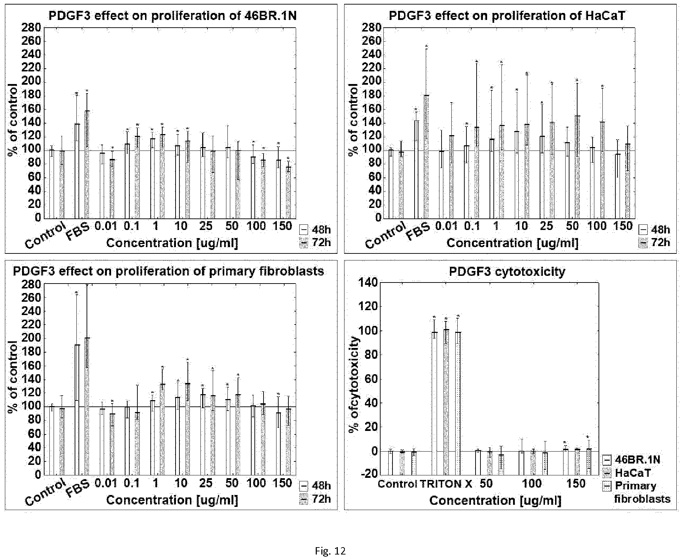

**Fig. 12** **-** presents graphs showing the effect of PDGF3 on proliferation (XTT test) of 46BR.1N fibroblasts, HaCaT keratinocytes and primary fibroblasts, and its cytotoxicity to these cells (LDH test). The graphs show a summary of results from 4 cycles of experiments (4 replicates in each, n = 16). * - statistically significant differences compared to the control (cells cultured in medium without serum), U-Mann Whitney's test (p <0.05, n = 16); FBS - positive control in the XTT test (cells grown in medium with 10% serum), TRITON X - positive control in the LDH test (cells incubated in medium with the addition of 1% triton X, maximum cytotoxicity).

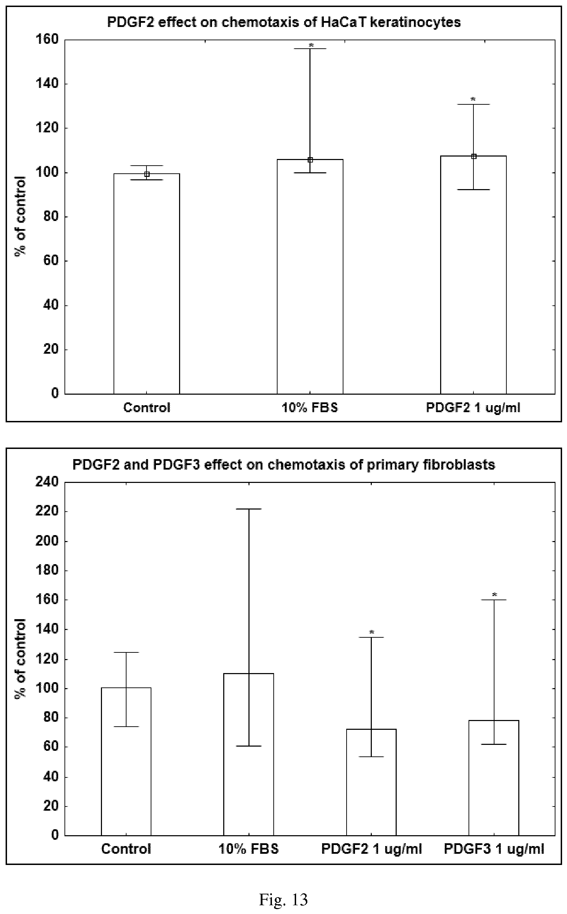

**Fig.13** - presents a graph showing chemotaxis of HaCaT keratinocytes (top). These are data from 4 cycles of experiments (n = 16). * - statistically significant differences compared to the control, U-Mann Whitney's test, p <0.05. The figure shows the effect of PDGF2 and PDGF3 (bottom) at a concentration of 1 µg / ml on chemotaxis of human primary dermal fibroblasts. These are data from 6 experimental cycles for PDGF2 and 5 for PDGF3. * - statistically significant differences, U Mann Whitney test, p <0.05.

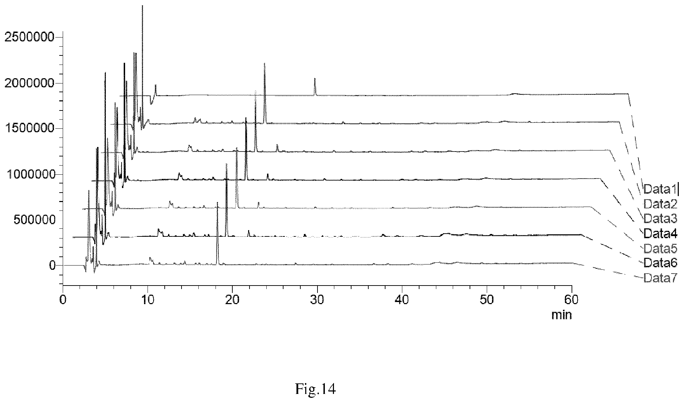

**Fig. 14** - PDGF2 peptide stability studies in human plasma. The peptide was incubated for 24 h. Its presence in the mixture was detected by HPLC with a PDA detector.
   Datal: PDGF2 0.1 mg/ml in water; Data2: plasma on its own after 24h; Data3: PDGF2 in plasma after 1h incubation; Data4: PDGF2 in plasma after 2h incubation; Data5: PDGF2 in plasma after 3h incubation; Data6: PDGF2 in plasma after 6h incubation; Data7: PDGF2 in plasma after 24h incubation.

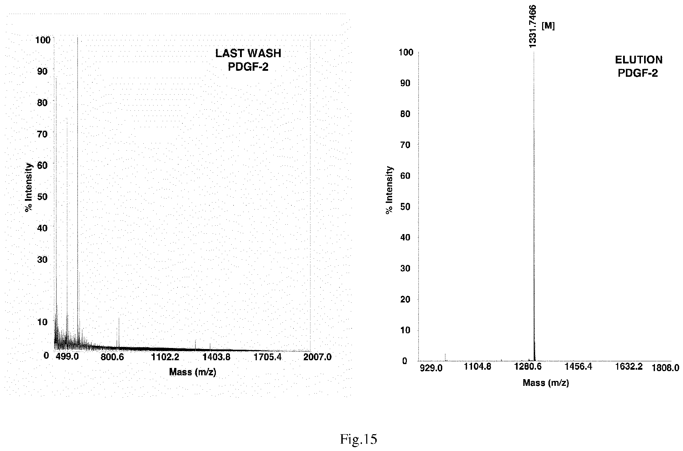

**Fig. 15****-** presents mass spectra of analyzed fractions of the PDGF2 peptide - last wash fraction (left) and elution fraction (right). In the last wash fraction, no signal was observed confirming that the excess of PDGF-2 peptide was removed. The presence of an m/z 1331,746 signal in the elution fraction is consistent with the mass of the PDGF2 peptide.

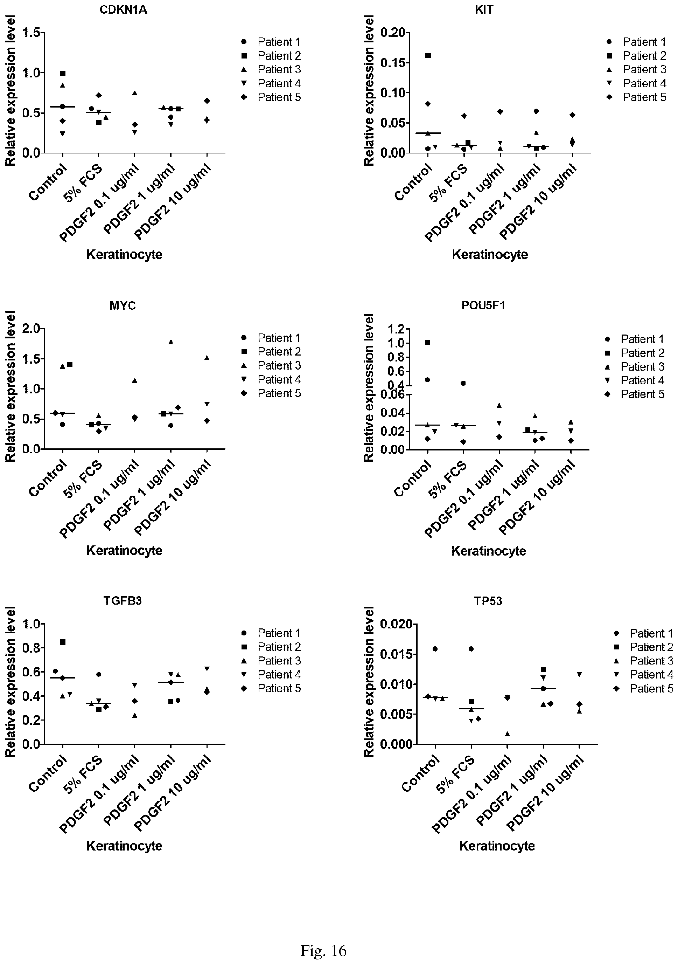

**Fig. 16** -presents graphs showing the effect of PDGF derivatives on the expression level of pluripotency genes and genes regulating the cell cycle (*CDKN1A, MYC, KIT, POU5F1, TGFB3, TP53)* in human primary keratinocyte cell line cultures isolated from five different patients. The reference genes are *AKTB* and *TBP.* Patients react to compounds in an individual way. There are no observable correlations and/ortrends in changes in expression level under the influence of the tested compounds

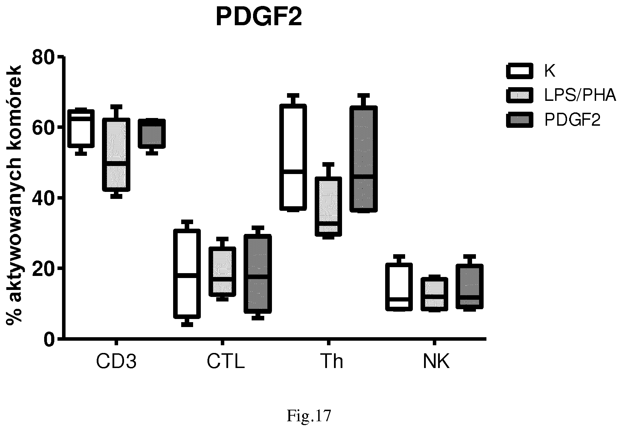

**Fig. 17** - presentsimmunogenicity analysis of PDGF2. This figure shows the percentage of immune cell activation in the presence of PDGF2, measured by flow cytometry. The activation level of T cells (CTL- cytotoxic T cells and Th - T helper cells) and NK cells was analyzed under the following conditions: control - unstimulated cells, cultured in pure medium; LPS/PHA - positive control, cells stimulated with lipopolysaccharide and phytohemagglutinin, PDGF2 - cells stimulated with the examined compound. The results are presented as the median (min-max).

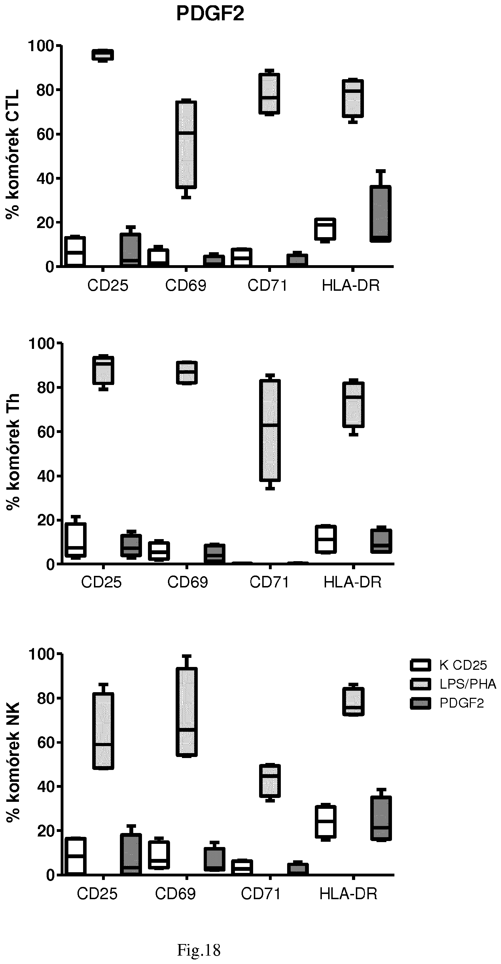

**Fig. 18** - presentsimmunogenicity analysis of PDGF2. This figure shows expression levels of surface activation markers (CD25, CD69, CD71, HLA-DR) on analyzed cell populations (CTL, Th, NK). The following stimulation conditions were used: control - unstimulated cells, cultured in pure medium; LPS/PHA - positive control, cells stimulated with lipopolysaccharide and phytohemagglutinin, PDGF2 - cells stimulated with the examined compound. The results are presented as the median (min-max).

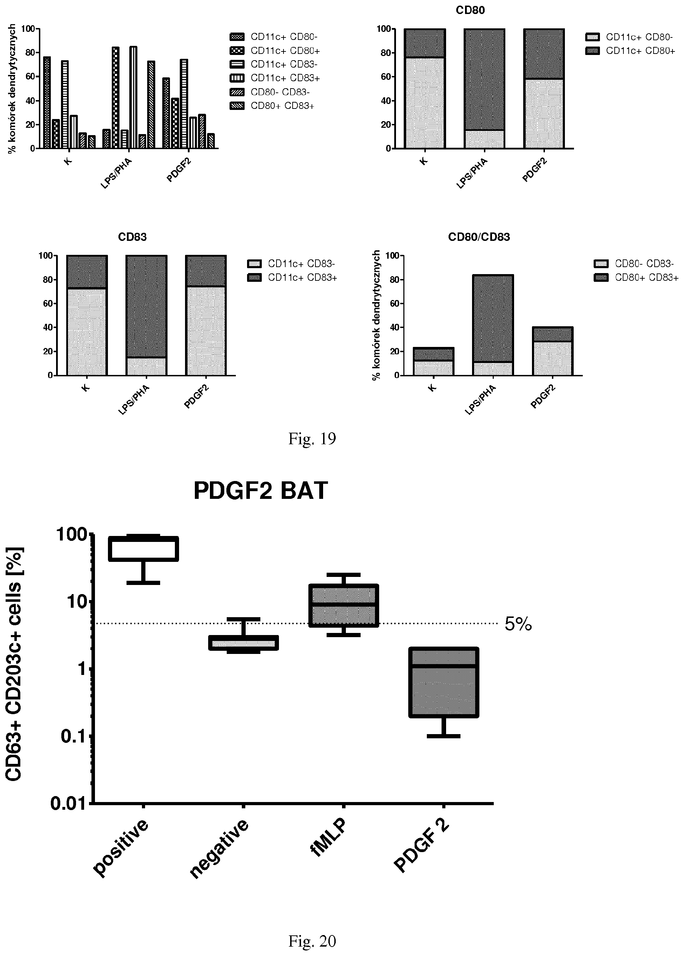

**Fig. 19** **-** presents **an** immunogenicity analysis of PDGF2. This figure shows activation level of dendritic cells cultured under the following conditions: control - unstimulated cells, cultured in pure medium; LPS/PHA - positive control, cells stimulated with lipopolysaccharide and phytohemagglutinin, PDGF2 - cells stimulated with the examined compound. The activity of dendritic cells was analyzed based on the expression level of the activation markers CD80 and CD83. Activated DC phenotypes: CD11c+ CD80+; CD11c+ CD83+; CD80+CD83+. The results are presented as the median (min-max).
**Fig. 20** **-** presents **an** immunogenicity analysis of PDGF2 - basophil activation test (BAT). The figure shows basophil activation in the presence of: negative control, positive control - basophil stimulating agent - fMLP, cells stimulated with PDGF2. The flow cytometry results are presented as the median (min-max).

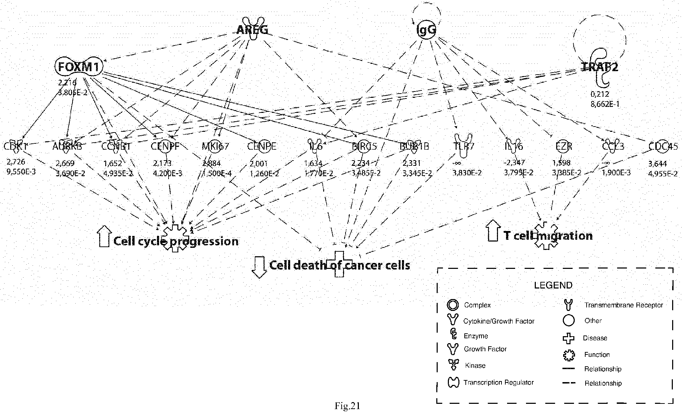

**Fig. 21** - presents a graph of the IPA Regulatory network with highest consistency score (6,414) for ASCs isolated from three patients (after RNA pooling) and stimulated with PDGF2 peptide (1 µg/ml). The values below the molecules correspond to log2-fold change of expression and p-value. A negative or positive value indicates decrease or increase of expression. In the bottom panel the arrow shows activation or inhibition of downstream effect.

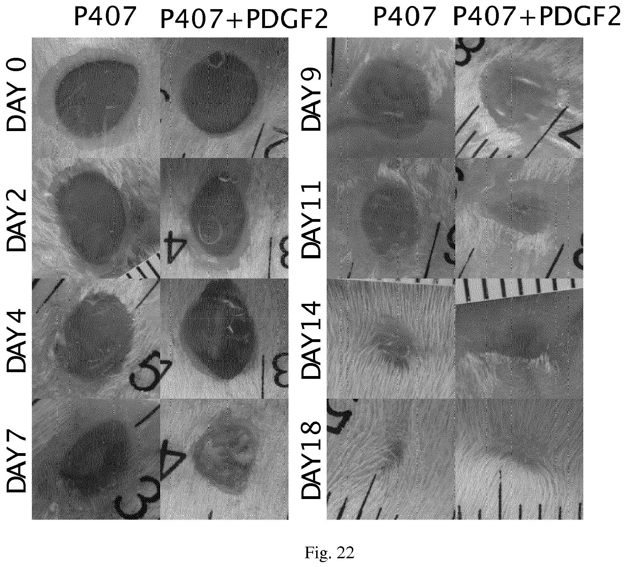

**Fig. 22** - shows representative photographs documenting the course of wound healing in the group receiving the P407 hydrogel with the PDGF2 peptide and in the control group with the P407 hydrogel alone. On day 11 there is an improvement in healing in the PDGF2 peptide group relative to the control. The agents were administered on the day of wounding and on days 1-4 post wounding, once a day, with 25 µl applied to each wound.

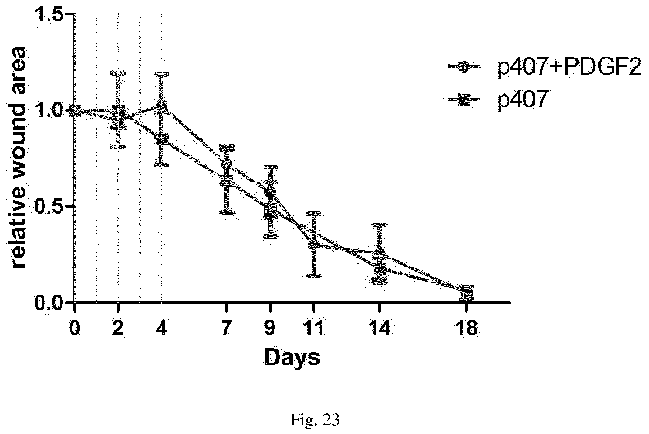

**Fig. 23** **-** shows a graph of the wound healing kinetics of the dorsal skin. Vertical, dashed lines indicate days in which the P407 hydrogel or the P407 hydrogel with the PDGF2 peptide (P407 + PDGF2) were administered. The graphs show the mean and standard deviations from the mean.

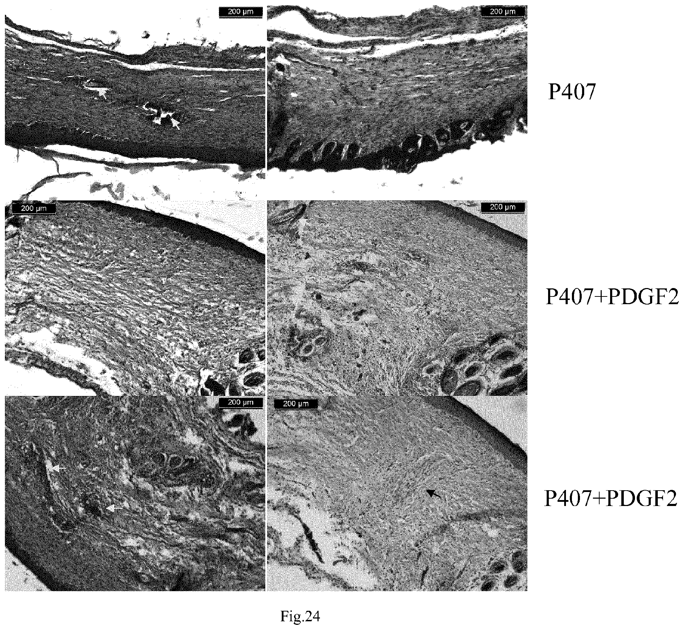

**Fig. 24** - presents pictures of histological staining. On the left, samples stained with hematoxylin and eosin, and on the right, Masson's staining. At the top are shown samples from control groups, and in the middle and at the bottom samples from the groups receiving PDGF2. The yellow arrows mark hair follicles and their ovules, while red arrows indicate glands. The black arrow indicates the area of cells differing from the surrounding fibroblasts.

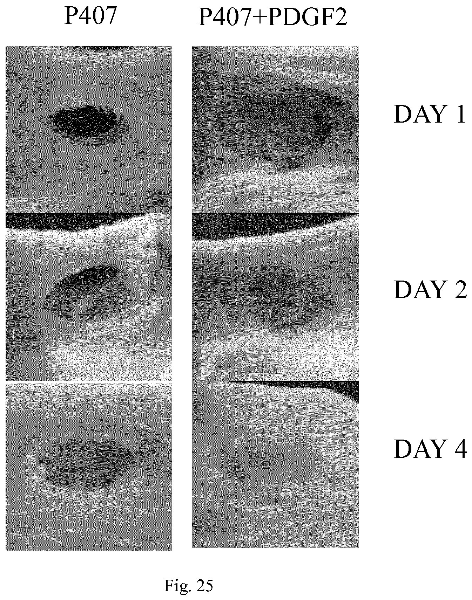

**Fig. 25** - presents representative pictures of membranes formed in the early days after wounding. In the groups receiving the P407 hydrogel with the PDGF2 peptide, the membranes cover the wound area faster and are also thicker and mechanically stronger. In these groups, the epidermis growth is accelerated under the influence of the PDGF2 peptide

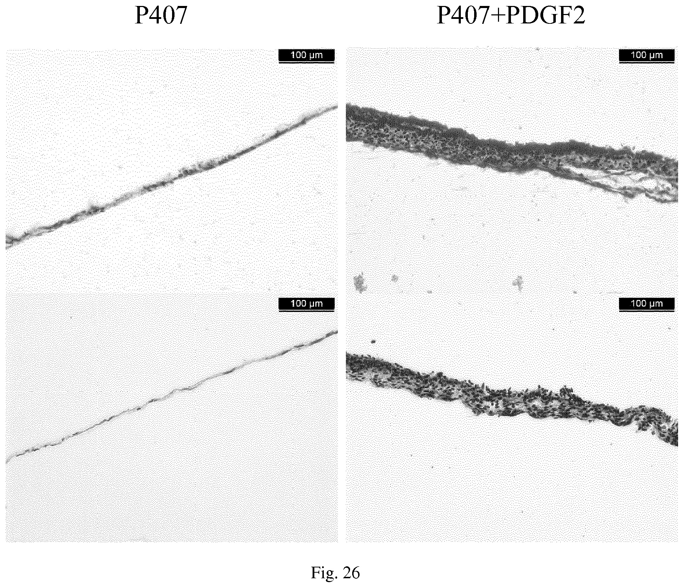

**Fig. 26** - presents pictures of histological samples of the epidermis, formed in the first four days of wound healing on the backs of mice. On the left are samples from the control group and on the right samples from groups of mice receiving the P407 hydrogel with the PDGF2 peptide. Samples on the top were stained with hematoxylin and eosin, while those on the bottom were stained with Masson's trichrome staining.

### EXAMPLES

The invention is further illustrated by the following non- limiting examples.

### I. CHEMICAL SYNTHESIS

### Example 1:

### Description of the synthesis procedure for the peptide with sequence: R¹K²I³E⁵I⁵V⁶R⁷K⁸K⁹P¹⁰I¹¹F¹²-NH₂(PDGF1)

### Synthesis:

Synthesis of the peptide with the sequence R¹K²I³E⁴I5V⁶R⁷K⁸K⁹P¹⁰I¹¹F¹²-NH₂ was performed on a solid support, according to Fmoc chemistry methodology,in a Liberty BlueTM automated microwave synthesizer (CEM Corporation). The TentaGel R RAM resin was used as a solid support with a capacity of 0.18 mmol/g.

Reagents used for the synthesis:
- DMF dried over molecular sieves type A3 and A4,
- 0.2 M solution of *N*^{α} -Fmoc-derived amino acids in DMF:
   o Fmoc-Val-OH,
   o Fmoc-Arg(Pbf)-OH,
   o Fmoc-Lys(Boc)-OH,
   o Fmoc-Glu(OtBu)-OH,
   o Fmoc-Ile-OH,
   o Fmoc-Pro-OH,
   o Fmoc-Phe-OH,
- 20% piperidine in DMF,
- 1 M Oxyma pure in DMF,
- 0.5 M DIC in DMF.

In the first step of the synthesis, the Fmoc-protected (9H-fluoren-9-yl-methyloxycarbonyl) amine group of the solid support was deprotected. The 20% solution of piperidine in DMF was used in this step simultaneously with irradiation of the mixture for 25 seconds with 125 W microwave power settings (temperature 70° C), then with a power of 30W for 65 seconds (temperature in the range 89-90° C). Next, the solid support was drained and washed 4 times with DMF. The first N-protected amino acid residue (Fmoc-Phe-OH) was attached to the resin with 0.5 M DIC solution in DMF (coupling reagent) and 1 M solution of Oxymy pure (racemization suppressor). The coupling reaction step was carried out with a 4-fold excess of amino acid residue (AA), calculated on the initial capacity of the solid support. This step was enhanced with microwave induction (170W) for 15 seconds (temperature 75° C), then for 110 seconds with a power of 30 W and temperature in the range of 89-90° C. The peptidyl resin was then drained and the 20% piperidine solution in DMF was added to deprotect the N-protected amine group of the first attached Phe residue (protected with Fmoc). This deprotection step was carried out using 125 W microwave radiation power for 25 seconds (temperature 70° C), then with 30 W for 65 seconds (temperature in the range of 89-90° C). The peptidyl resin was subsequently drained and washed 4 times with DMF. The remaining amino acids in the sequence were further coupled analogously to the above description, and according to Fig.1. After completion of the synthesis, the peptidyl resin was washed 4 times with DMF, then 3 times with MeOH and left overnight to dry in a vacuum desiccator.

### Peptide cleavage from the solid support:

The peptide was cleaved from the solid support, along with removal of protecting groups from AA side chains, using the mixture TFA/TIPSI/H₂O (92:4:4, v/v/v). The reaction was carried out for two hours on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C). A white precipitate was obtained and centrifuged in a centrifuge tube for 15 minutes (4500 x g). The supernatant was decanted and the pellet was treated with another portion of Et₂O. The precipitate was washed 3 times as described above, then dried in a vacuum desiccator. The obtained raw product was dissolved in water and freeze-dried.

### Purification:

The compound was purified using high performance liquid chromatography. The crude product of the synthesis was dissolved in water and injected onto a Jupiter® Proteo C12 semipreparative column (Phenomenex) with dimensions 21.2 mm x 250 mm, 90 Å, 4 µm. The chromatographic separation was carried out in a linear gradient of 5-100% B over 180 minutes with eluents: A = 0.1% TFA in H₂O and B = 0.1% TFA, 80% ACN in H₂O. The eluent flow rate was 13 ml/min, UV detection at X = 223 nm.

### Characteristics of the product - HPLC and mass spectrometry:

The crude product was subjected to chromatographic analysis using high performance reverse phase liquid chromatography (RP-HPLC). Chromatographic column: Jupiter 4 µmProteo 90 Å, 250 x 4.6 mm. Eluents: A = 0.1% TFA in H₂Owater, B = 0.1% TFA, 80% ACN in water, flow rate 1 ml/min, UV detection at λ = 223 nm, linear gradient of 5% B → 100% B over 30 minutes, Rₜ = 12.8 minutes. The calculated molecular weight of the compound was confirmed with a MALDI-TOF mass spectrometer (Bruker Daltonics). Theoretical average molecular weight of the compound: 1525.9 Da, obtained m/z: 1526.9 ([M + H]⁺).

### Exchange of trifluoroacetic counterion with acetate ion:

Exchange of the trifluoroacetic counterion with an acetate ion was performed on a Strata X-C 33U Polymeric Strong Cation column according to the procedure:
- washing the column with methanol, then with a 0.1 M solution of acetic acid in water,
- dissolving the compound in water and applying to the equilibrated column,
- binding the compound to the bed of the column, with progress being monitored by RP-HPLC,
- if the compound has bound to the column, then washing with 0.1 M aqueous acetic acid,
- eluting the compound with 1 M aqueous ammonium acetate in 50% MeOH (v/v).
The eluate was evaporated with vacuum evaporator and lyophilized. The lyophilisate was redissolved in water and re-lyophilized to remove remaining ammonium acetate.

### Example 2:

### Description of the synthesis procedure for the peptide with sequence: AC-R¹K²I³E⁴I⁵V⁶ R⁷K⁸K⁹P¹⁰I¹¹F¹²-NH₂(Ac-PDGFI)

### Synthesis:

Synthesis of the peptide with the sequence Ac-R¹K²I³E⁴I⁵V⁶R⁷K⁸K⁹P¹⁰I¹¹F¹²-NH₂ was performed on a solid support, according to Fmoc chemistry methodology, in a Liberty BlueTM automated microwave synthesizer (CEM Corporation). TentaGel R RAM resin was used as the solid support, with a capacity of 0.18 mmol/g.

Reagents used for the synthesis:
- DMF dried over molecular sieves type A3 and A4,
- 0.2 M solution of *N*^{α} -Fmoc-derived amino acids in DMF:
   ∘ Fmoc-Val-OH,
   ∘ Fmoc-Arg(Pbf)-OH,
   ∘ Fmoc-Lys(Boc)-OH,
   ∘ Fmoc-Glu(OtBu)-OH,
   ∘ Fmoc-Ile-OH,
   ∘ Fmoc-Pro-OH,
   ∘ Fmoc-Phe-OH,
- 20% piperidine in DMF,
- 1 M Oxyma pure in DMF,
- 5% acetic anhydride solution, 2% DIPEA in DMF,
- 0.5 M DIC in DMF.

In the first step of the synthesis, the Fmoc-protected (9H-fluoren-9-yl-methyloxycarbonyl) amine group of the solid support was deprotected. The 20% solution of piperidine in DMF was used in this step simultaneously with irradiation of the mixture for 25 seconds with 125 W microwave power settings (temperature 70° C), then with a power of 30 W for 65 seconds (temperature in the range 89-90° C). The solid support was then drained and washed 4 times with DMF. The first N-protected amino acid residue (Fmoc-Phe-OH) was attached to the resin with 0.5 M DIC solution in DMF (coupling reagent) and 1 M solution of Oxymy pure (racemization suppressor). The coupling reaction step was carried out with a 4-fold excess of amino acid residue (AA), calculated on the initial capacity of the solid support. This step was enhanced with microwave induction (170 W) for 15 seconds (temperature 75° C), then for 110 seconds with a power of 30 W and a temperature in the range of 89-90° C. The peptidyl resin was then drained and the 20% piperidine solution in DMF was added to deprotect the N-protected amine group of the first attached Phe residue (protected with Fmoc). This deprotection step was carried out using 125 W microwave radiation power for 25 seconds (temperature 70° C), then with 30 W for 65 seconds (temperature in the range of 89-90° C). The peptidyl resin was subsequently drained and washed 4 times with DMF. The remaining amino acids in the sequence were further coupled analogously to the above description, and according to Fig.2. In the last step of the synthesis, the peptidyl resin was drained and washed 4 times with DMF, followed by capping the N-terminal amine group of the last AA with an acetyl group (Ac). The mixture of 5% Ac₂O, 2% DIPEA in DMF (5 ml) was added to the peptidyl resin. The reaction was carried out in the presence of microwave radiation (30 W power) for 5 minutes and 50° C. After completion of the synthesis, the peptidyl resin was washed 4 times with DMF, then 3 times with MeOH and left overnight to dry in a vacuum desiccator.

### Peptide cleavage from the solid support:

The peptide was cleaved from the solid support, along with removal of protecting groups from AA side chains, using the mixture TFA/TIPSI/H₂O (92:4:4, v/v/v). The reaction was carried out for two hours on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C). A white precipitate was obtained and centrifuged in a centrifuge tube for 15 minutes (4500 x g). The supernatant was decanted and the pellet was treated with another portion of Et₂O. The precipitate was washed 3 times as described above, then dried in a vacuum desiccator. The obtained raw product was dissolved in water and freeze-dried.

### Purification:

The compound was purified using high performance liquid chromatography. The crude product of the synthesis was dissolved in water and injected onto a Jupiter® Proteo C12 semipreparative column (Phenomenex) with dimensions 21.2 mm x 250 mm, 90 Å, 4 µm. The chromatographic separation was carried out in a linear gradient of 5-100% B over 180 minutes with eluents: A = 0.1% TFA in H₂O and B = 0.1% TFA, 80% ACN in H₂O. The eluent flow rate was 13 ml/min, UV detection at λ = 223 nm.

### Characteristics of the product - HPLC and mass spectrometry:

The crude product was subjected to chromatographic analysis using high performance reverse phase liquid chromatography (RP-HPLC). Chromatographic column: Kinetex 2,6 µm 100 Å, 100 x 2.1 mm. Eluents: A = 0.1% TFA in H₂O, B = 0.1% TFA, 80% ACN in water, flow rate 0.5 ml/min, UV detection at λ = 223 nm, linear gradient of 5% B → 100% B over 10 minutes, temperature of oven 40° C, Rₜ = 3.67 minutes. The calculated molecular weight of the compound was confirmed with a MALDI-TOF mass spectrometer (Bruker Daltonics). Theoretical average molecular weight of the compound: 1568.0 Da, obtained m/z: 1568.9 ([M + H]⁺).

### Exchange of trifluoroacetic counterion with acetate ion:

Exchange of the trifluoroacetic counter ion with an acetate ion was performed on a Strata X-C 33U Polymeric Strong Cation column according to the procedure:
- washing the column with methanol, then with a 0.1 M solution of acetic acid in water,
- dissolving the compound in water and applying to the equilibrated column,
- binding the compound to the bed of the column, with progress being monitored by RP-HPLC,
- if the compound has bound to the column, then washing with 0.1 M aqueous acetic acid,
- eluting the compound with 1 M aqueous ammonium acetate in 50% MeOH (v/v).
The eluate was evaporated with a vacuum evaporator and lyophilized. The lyophilisate was redissolved in water and re-lyophilized to remove remaining ammonium acetate.

### Example 3:

### Description of the synthesis procedure for the peptide with sequence: R¹L²I³D⁴R⁵T⁶N⁷A⁸N⁹F¹⁰L¹¹-NH₂(PDGF2)

### Synthesis:

Synthesis of the peptide with the sequence R¹L²I³D⁴R⁵T⁶N⁷A⁸N⁹F¹⁰L¹¹-NH₂ was performed on a solid support, according to Fmoc chemistry methodology, in a Liberty BlueTM automated microwave synthesizer (CEM Corporation). TentaGel R RAM resin was used as a solid support with a capacity of 0.18 mmol/g.

Reagents used for the synthesis:
- DMF dried over molecular sieves type A3 and A4,
- 0.2 M solution of *N*^{α} -Fmoc-derived amino acids in DMF:
   ∘ Fmoc-Ala-OH,
   ∘ Fmoc-Arg(Pbf)-OH,
   ∘ Fmoc-Asn(Trt)-OH,
   ∘ Fmoc-Asp(OtBu)-OH,
   ∘ Fmoc-Ile-OH,
   ∘ Fmoc-Leu-OH,
   ∘ Fmoc-Phe-OH,
   ∘ Fmoc-Thr(tBu)-OH,
- 20% piperidine in DMF,
- 1 M Oxyma pure in DMF,
- 0.5 M DIC in DMF.

In the first step of the synthesis, the Fmoc-protected (9H-fluoren-9-yl-methyloxycarbonyl) amine group of the solid support was deprotected. The 20% solution of piperidine in DMF was used in this step simultaneously with irradiation of the mixture for 25 seconds with 125 W microwave power settings (temperature 70° C), then with a power of 30 W for 65 seconds (temperature in the range 89-90° C). The solid support was then drained and washed 4 times with DMF. The first N-protected amino acid residue (Fmoc-Leu-OH) was attached to the resin with 0.5 M DIC solution in DMF (coupling reagent) and 1 M solution of Oxymy pure (racemization suppressor). The coupling reaction step was carried out with a 4-fold excess of amino acid residue (AA) calculated on the initial capacity of the solid support. This step was enhanced with microwave induction (170 W) for 15 seconds (temperature 15° C), then for 110 seconds with a power of 30 W and temperature in the range of 89-90° C. The peptidyl resin was then drained and the 20% piperidine solution in DMF was added to deprotect the N-protected amine group of the first attached Leu residue (protected with Fmoc). This deprotection step was carried out using 125 W microwave radiation power for 25 seconds (temperature 70° C), then with 30 W for 65 seconds (temperature in the range of 89-90° C). The peptidyl resin was then drained and washed 4 times with DMF. The remaining amino acids in the sequence were further coupled analogously to the above description, and according to Fig.3. After completion of the synthesis, the peptidyl resin was washed 4 times with DMF, then 3 times with MeOH and left overnight to dry in a vacuum desiccator.

### Peptide cleavage from the solid support:

The peptide was cleaved from the solid support, along with removal of protecting groups from AA side chains, using the mixture TFA/TIPSI/H₂O (92:4:4, v/v/v). The reaction was carried out for two hours on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C). A white precipitate was obtained and centrifuged in a centrifuge tube for 15 minutes (4500 x g). The supernatant was decanted and the pellet was treated with another portion of Et₂O. The precipitate was washed 3 times as described above, then dried in a vacuum desiccator. The obtained raw product was dissolved in water and freeze-dried.

### Purification:

The compound was purified using high performance liquid chromatography. The crude product of the synthesis was dissolved in water and injected onto a Jupiter® Proteo C12 semipreparative column (Phenomenex) with dimensions 21.2 mm x 250 mm, 90 Å, 4 µm. The chromatographic separation was carried out in a linear gradient of 5-100% B over 180 minutes with eluents: A = 0.1% TFA in H₂O and B = 0.1% TFA, 30% ACN in H₂O. The eluent flow rate was 14 ml/min, UV detection at λ = 223 nm.

### Characteristics of the product - HPLC and mass spectrometry:

The crude product was subjected to chromatographic analysis using high performance reverse phase liquid chromatography (RP-HPLC). Chromatographic column: Jupiter 4 µmProteo 90 Å, 250 x 4.6 mm. Eluents: A = 0.1% TFA in H₂O, B = 0.1% TFA, 80% ACN in water, flow rate 1 ml/min, UV detection at λ = 223 nm, linear gradient of 5% B → 100% B over 60 minutes, Rₜ = 19.9 minutes. The calculated molecular weight of the compound was confirmed with MALDI-TOF mass spectrometer (Bruker Daltonics). Theoretical average molecular weight of the compound: 1331.5 Da, obtained m/z: 1332.6 ([M + H]⁺).

### Exchange of trifluoroacetic counterion with acetate ion:

Exchange of the trifluoroacetic counter ion with acetate ion was performed on a Strata X-C 33U Polymeric Strong Cation column according to the procedure:
- washing the column with methanol, then with a 0.1 M solution of acetic acid in water,
- dissolving the compound in water and applying to the equilibrated column,
- binding the compound to the bed of the column, with progress being monitored by RP-HPLC,
- if the compound has bound to the column, washing with 0.1 M aqueous acetic acid,
- eluting the compound with 1 M aqueous ammonium acetate in 50% MeOH (v/v).
The eluate was evaporated with a vacuum evaporator and lyophilized. The lyophilisate was redissolved in water and re-lyophilized to remove remaining ammonium acetate.

### Example 4:

### Description of the synthesis procedure for the peptide with sequence:

PEG2¹R²L³I⁴D⁵R⁶T⁷N⁸A⁹N¹⁰F¹¹L¹²
(PDGF2-HTT)

### Synthesis:

Synthesis of the PDGF2-HTT peptide was performed on a solid support, according to Fmoc chemistry methodology, in a Liberty BlueTM automated microwave synthesizer (CEM Corporation). Cl-TCP(Cl) ProTide (CEM Corporation) resin was used as a solid support with a capacity of 0.4 mmol/g.

Reagents used for the synthesis:
- DMF dried over molecular sieves type A3 and A4,
- 0.2 M solution of *N*^{α} -Fmoc-derived amino acids in DMF:
   ∘ Fmoc-Ala-OH,
   ∘ Fmoc-Arg(Pbf)-OH,
   ∘ Fmoc-Asn(Trt)-OH,
   oFmoc-Asp(OtBu)-OH,
   ∘ Fmoc-Ile-OH,
   ∘ Fmoc-Leu-OH,
   ∘ Fmoc-PEG2-OH,
   ∘ Fmoc-Phe-OH,
   ∘ Fmoc-Thr(tBu)-OH,
- 20% piperidine in DMF,
- 1 M Oxyma pure in DMF,
- 0.5 M DIC in DMF.

The first amino acid (Fmoc-Leu- OH) was attached to the solid support using 0.2 M of the N-protected amino acid solution (Fmoc-Leu-OH) in 1 M DIPEA + 0.125 M KI in DMF. The mixture was subjected to microwave irradiation at 75W for 60 seconds at 80° C, then at 20W for 540 seconds (temperature 90° C). The peptidyl resin was drained and washed 5 times with DMF. In the next step, the Fmoc-protected (9H-fluoren-9-yl-methyloxycarbonyl) amine group of the first resin-attached AA (Fmoc-Leu-OH) was deprotected. The 20% solution of piperidine in DMF was used in this step simultaneously with irradiation of the mixture for 25 seconds with 125 W microwave power settings (temperature 70° C), then with a power of 30 W for 65 seconds (temperature in the range 89-90° C). The solid support was then drained and washed 4 times with DMF. The next N-protected amino acid (Fmoc-Phe-OH) was coupled with 0.5 M DIC solution in DMF (coupling reagent) and 1 M solution of Oxymy pure (racemization suppressor). The coupling reaction step was carried out with a 4-fold excess of amino acid residue, calculated on the initial capacity of the solid support. This step was enhanced with microwave induction (170W) for 15 seconds (temperature 75° C), then for 110 seconds with a power of 30 W and temperature in the range of 89-90° C. The peptidyl resin was then drained and the 20% piperidine solution in DMF was added to deprotect the N-protected amine group of the first attached Leu residue (protected with Fmoc). This deprotection step was carried out using 125 W microwave radiation power for 25 seconds (temperature 70° C), then at 30 W for 65 seconds (temperature in the range of 89-90° C). The peptidyl resin was then drained and washed 4 times with DMF. The rest of the amino acids in the sequence were further coupled analogously to the above description, and according to Fig.4. After completion of the synthesis, the peptidyl resin was washed 4 times with DMF, then 3 times with MeOH and left overnight to dry in a vacuum desiccator.

### Peptide cleavage from the solid support, and cyclization:

The peptide was cleaved from the solid support with 1% TFA in dichloromethane (v/v) while leaving the side chain protecting groups of all residues. The reaction was left for one hour on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C). A white precipitate was obtained and centrifuged in a centrifuge tube for 15 minutes (4500 x g). The supernatant was decanted and the pellet was treated with another portion of Et₂O. The precipitate was washed 3 times as described above, then dried in a vacuum desiccator. The crude product was cyclized by formation of a peptide bond between the amino group at the N-terminus and the carboxyl group at the C-terminus. The reactants were placed in two separatory funnels: the first contained 0.1 mmol of the compound dissolved in 60 ml of DMF, while the second contained 0.3 mmol HATU in 60 ml of DMF. The reagents from both separatory funnels were simultaneously added into a round bottom flask containing 0.1 mmol HATU in 150 ml of DMF and provided with a magnetic stirrer. The rate of addition was about 100 µl/minute and the reaction was continued for about 11 h. After this step, the solvent was evaporated with a vacuum evaporator equipped with an oil pump. The oily remains, together with the precipitate, were dissolved in 20 ml of 70% ACN solution with addition of 0.07% TFA. A small amount of undissolved precipitate was separated by filtration. The filtrate was diluted with water to a volume of approximately 100 ml. The compound was pre-purified by HPLC on a Jupiter® Proteo C12 (Phenomenex) column with dimensions 21.2 mm x 250 mm, 90 Å, 4 µm. The chromatographic separation was carried out in a linear gradient of 5-100% B over 180 minutes with eluents: A = 0.1% TFA in H₂O and B = 0.1% TFA, 80% isopropanol in H₂O. The eluent flow rate was 14 ml/min, UV detection at λ = 223 nm. Pure fractions were collected and lyophilized. Cleavage of remaining side chain protecting groups of amino acids in the peptide was carried out by treating the cyclic form with the mixture TFA/TIPSI/H₂O (94:3:3, v/v/v). The reaction was carried out for two hours on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C). A white precipitate was obtained and centrifuged in a centrifuge tube for 15 minutes (4500 x g). The supernatant was decanted and the pellet was treated with another portion of Et₂O. The precipitate was washed 3 times as described above, then dried in a vacuum desiccator. The obtained raw product was dissolved in water and freeze-dried.

### Purification:

The compound was purified using high performance liquid chromatography. The crude product of the synthesis was dissolved in water and injected into a Jupiter® Proteo C12 semipreparative column (Phenomenex) with dimensions 21.2 mm x 250 mm, 90 Å, 4 µm. The chromatographic separation was carried out in a linear gradient of 5-100% B over 180 min with eluents: A = 0.1% TFA in H₂O and B = 0.1% TFA, 35% ACN in H₂O. The eluent flow rate was 13 ml/min, UV detection at λ = 223 nm.

### Characteristics of the product - HPLC and mass spectrometry:

The crude product was subjected to chromatographic analysis using high performance reverse phase liquid chromatography (RP-HPLC). Chromatographic column: Jupiter 4 µmProteo 90 Å, 250 x 4.6 mm. Eluents: A = 0.1% TFA in H₂O, B = 0.1% TFA, 80% ACN in water, flow rate 1 ml/min, UV detection at λ = 223 nm, liner gradient of 5% B → 100% B over 30 minutes, Rₜ = 13.8 minutes. The calculated molecular weight of the compound was confirmed with MALDI-TOF mass spectrometer (Bruker Daltonics). Theoretical average molecular weight of the compound: 1459.9 Da, obtained m/z: 1460.8 ([M+H]⁺).

### Exchange of trifluoroacetic counterion with acetate ion:

Exchange of the trifluoroacetic counterion with acetate ion was performed on a Strata X-C 33U Polymeric Strong Cation column according to the procedure:
- washing the column with methanol, then with a 0.1 M solution of acetic acid in water,
- dissolving the compound in water and applying to the equilibrated column,
- binding the compound to the bed of the column, with progress being monitored by RP-HPLC,
- if the compound has bound to the column, then washing with 0.1 M aqueous acetic acid,
- eluting the compound was with 1 M aqueous ammonium acetate in 50% MeOH (v/v).
The eluate was evaporated with vacuum evaporator and lyophilized. The lyophilisate was redissolved in water and re-lyophilized to remove remainingammonium acetate.

### Example 5:

### Description of the peptide synthesis procedure for the peptide with sequence:

Ac-R¹L²I³D⁴R⁵T⁶N⁷A⁸N⁹F¹⁰L¹¹-G¹²G¹³G¹⁴-A¹⁵A¹⁶P¹⁷V¹⁸-G¹⁹G²⁰G²¹-
R²²L²³I²⁴D²⁵R²⁶T²⁷N²⁸A²⁹N³⁰F³¹L³²-NH₂(PDGF2-NE)

Reagents used for the synthesis:
- DMF dried over molecular sieves type A3 and A4,
- 0.2 M solution of *N*^{α} -Fmoc-derived amino acids in DMF:
   ∘ Fmoc-Ala-OH,
   ∘ Fmoc-Arg(Pbf)-OH,
   ∘ Fmoc-Asn(Trt)-OH,
   oFmoc-Asp(OtBu)-OH,
   ∘ Fmoc-Glu(OtBu)-OH,
   ∘ Fmoc-Gly-OH,
   ∘ Fmoc-Ile-OH,
   ∘ Fmoc-Leu-OH,
   ∘ Fmoc-Lys(Boc)-OH,
   ∘ Fmoc-Lys(Mtt)-OH,
   ∘ Fmoc-Phe-OH,
   ∘ Fmoc-Pro-OH,
   ∘ Fmoc-Thr(tBu)-OH,
   ∘ Fmoc-Val-OH,
- 20% piperidine in DMF,
- 1 M Oxyma pure in DMF,
- 2% DIPEA in DMF,
- 5% acetic anhydride solution, 2% DIPEA in DMF,
- 1% TFA w DCM,
- 0.5 M DIC in DMF.

### Synthesis:

Synthesis of the peptide with the sequence Ac-R¹L²I³D⁴R⁵T⁶N⁷A⁸N⁹F¹⁰L¹¹-G¹²G¹³G¹⁴-A¹⁵A¹⁶P¹⁷V¹⁸-G¹⁹G²⁰G²¹-R²²L²³I²⁴D²⁵R²⁶T²⁷N²⁸A²⁹N³⁰F³¹L³²-NH₂ was performed on a solid support, according to Fmoc chemistry methodology, in an Activo-P11 (Activotec Corp.) automated peptide synthesizer. TentaGel R RAM resin was used as a solid support with a capacity of 0.18 mmol/g.

In the first step of the synthesis, the Fmoc-protected (9H-fluoren-9-yl-methyloxycarbonyl) amine group of the solid support was deprotected with the 20% solution of piperidine in DMF at 30° C. The solid support was then drained and washed 4 times with DMF. The first N-protected amino acid residue (Fmoc-Leu-OH) was attached to the resin with 0.5 M DIC solution in DMF (coupling reagent) and 1 M solution of Oxymy pure (racemization suppressor). The coupling reaction step was carried out with a 3-fold excess of amino acid residue, calculated on the initial capacity of the solid support, at a temperature of 50° C. The peptidyl resin was then drained and the 20% piperidine solution in DMF was added to deprotect the N-protected amine group of the first attached residue. The peptidyl resin was then drained and washed 4 times with DMF. The rest of the amino acids in the sequence were further coupled analogously to the above description. After completion of the synthesis, the peptidyl resin was washed 4 times with DCM, then 3 times with MeOH, 3 times with Et₂O and left overnight to dry in a vacuum desiccator.

### Peptide cleavage from the solid support:

The peptide was cleaved from the solid support, along with removal of protecting groups from the amino acid side chains, using the mixture TFA/H₂O/Phenol/TIPSI/EDT/Thioanisole (81.5:5:5:1:2,5:5, v/v/v/v/v/v). The reaction was carried out for 3 hours on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C) to give a white precipitate. This was centrifuged in a centrifuge tube for 15 minutes (4500 x g). The supernatant was decanted and the pellet was treated with another portion of Et₂O. The precipitate was washed 3 times as described above, then dried in a vacuum desiccator. The obtained raw product was dissolved in water and freeze-dried.

### Purification:

The compound was purified using high performance liquid chromatography. The crude product of the synthesis was dissolved in water and injected onto a Kromasil C8 semipreparative column with dimensions 10.0 mm x 250 mm, 300 Å, 5 µm. The chromatographic separation was carried out in a linear gradient of 25-80% B over 30 minutes, then 80-100% B over 180 min with eluents: A = 0.2 M aqueous ammonium acetate (pH 4.75) and B = 20% MeOH in 0.2 M aqueous ammonium acetate (pH 4.75). The eluent flow rate was 5 ml/min, UV detection at λ = 223 nm.

### Characteristics of the product - HPLC and mass spectrometry:

The crude product was subjected to chromatographic analysis using high performance reverse phase liquid chromatography (RP-HPLC). [Chromatographic column: Kinetex 2.6 µm 100 Å, 100 x 2,1 mm. Eluents: A = 0.1% TFA in H₂O, B = 0.1% TFA, 80% ACN in water, flow rate 0.5 ml/min, UV detection at λ = 223 nm, liner gradient of 5% B → 100% B over 15 minutes, temperature of oven 40° C, Rₜ = 4.78 minutes]. The calculated molecular weight of the compound was confirmed using a LC MS IT-TOF (Shimadzu) device. Theoretical average molecular weight of the compound: 3366.79 Da, obtained m/z: 842.80 [M+4H]⁴⁺, 674.44 [M+5H]⁵⁺ (After deconvolution 3366.17 Da). [Chromatographic column Kromasil C8 5 µm 100 Å, 250 x 1.0 mm. Eluents: A = 0.2% formic acid in H₂O, B = 0.2 % formic acid, 100%, ACN in H₂O, flow rate 0,08 ml/min, detector ELSD and PDA, liner gradient of 5% B → 100% B over 25 min, Rₜ= 16.26 min.]

### Example 6:

### Description of the synthesis procedure for peptides with sequence:

Ac-R¹K²I³E⁴I⁵V⁶R⁷K⁸K⁹P¹⁰I¹¹F¹²-NH₂
L²³F²²N²¹A²⁰N¹⁹T¹⁸R¹⁷D¹⁶I¹⁵L¹⁴R¹³
(PDGF3)

### Synthesis:

Synthesis of the peptide with the sequence Ac-R¹K²I³E⁴I⁵V⁶R⁷K⁸K⁹P¹⁰I¹¹F¹²-NH₂ was performed on a solid support, according to Fmoc chemistry methodology,using a Liberty BlueTM automated microwave synthesizer (CEM Corporation). TentaGel R RAM resin was used as solid support with a capacity of 0.18 mmol/g.

Reagents used for the synthesis:
- DMF dried over molecular sieves type A3 and A4,
- 0.2 M solution of *N*^{α} -Fmoc-derived amino acids in DMF:
   ∘ Fmoc-Ala-OH,
   ∘ Fmoc-Arg(Pbf)-OH,
   ∘ Fmoc-Asn(Trt)-OH,
   oFmoc-Asp(OtBu)-OH,
   ∘ Fmoc-Glu(OtBu)-OH,
   ∘ Fmoc-Ile-OH,
   ∘ Fmoc-Leu-OH,
   ∘ Fmoc-Lys(Boc)-OH,
   oFmoc-Lys(Mtt)-OH,
   ∘ Fmoc-Phe-OH,
   ∘ Fmoc-Pro-OH,
   ∘ Fmoc-Thr(tBu)-OH,
   ∘ Fmoc-Val-OH,
- 20% piperidine in DMF,
- 1 M Oxyma pure in DMF,
- 2% DIPEA in DMF,
- 5% acetic anhydride solution, 2% DIPEA in DMF,
- 1 % TFA w DCM,
- 0.5 M DIC in DMF.

In the first step of the synthesis, the Fmoc-protected (9H-fluoren-9-yl-methyloxycarbonyl) amine group of the solid support was deprotected. The 20% solution of piperidine in DMF was used in this step simultaneously with irradiation of the mixture for 25 seconds with 125 W microwave power settings (temperature 70° C), then with a power of 30W for 65 seconds (temperature in the range 89-90° C). The solid support was then drained and washed 4 times with DMF. The first N-protected amino acid residue (Fmoc-Phe-OH) was attached to the resin with 0.5 M DIC solution in DMF (coupling reagent) and 1 M solution of Oxymy pure (racemization suppressor). The coupling reaction step was carried out with a 4-fold excess of amino acid residue, calculated on the initial capacity of the solid support. This step was enhanced with microwave induction (170 W) for 15 seconds (temperature 75° C), then for 110 seconds with a power of 30 W and temperature in the range of 89-90° C. The peptidyl resin was then drained and the 20% piperidine solution in DMF was added to deprotect the N-protected amine group of the first attached Phe residue (protected with Fmoc). This deprotection step was carried out using 125 W microwave radiation power for 25 seconds (temperature 70° C), then at 30 W for 65 seconds (temperature in the range of 89-90° C). The peptidyl resin was then drained and washed 4 times with DMF. The remaining amino acids in the sequence were further coupled analogously to the above description, and according to Fig. 6a.

In the last step in the synthesis of fragment 1, the N-terminus Fmoc protecting group was removed followed by capping the N-terminal amine group of the last amino acid with an acetyl group. The mixture of: 5% Ac₂0, 2% DIPEA in DMF (5 ml) was added to the peptidyl resin. The reaction was carried out in the presence of microwave radiation (30 W power) for 5 minutes and 50 ° C. The resin was drained and washed 4 times with DMF.

In the next step of the synthesis, selective deprotection of the side chain of the Lys² residue protected with MTT was performed. The peptidyl resin was transferred to a glass funnel and a cycle of 20washes with 5 ml of 1% TFA in DCM was performed. The mixture was allowed to stand for 2 minutes at room temperature in each cycle, followed by draining and addition of a fresh portion of the mixture. The peptidyl resin was then washed 3 times with 10 ml of 2% of DIPEA in DMF, then washed 4 times with DMF and transferred back to the automated microwave peptide synthesizer. The synthesis was continued analogously to the above description, and according to Fig.6b, though in the stages to couple the Leu ²³ and Phe²² residues the acylation step was repeated without deprotection between the cycles. The synthesis scheme of the fragment 2 is presented in Fig.6b. After completion of the synthesis, the peptidyl resin was washed 4 times with DMF, then 3 times with MeOH and left overnight to dry in a vacuum desiccator.

### Peptide cleavage from the solid support:

The peptide was cleaved from the solid support, along with removal of protecting groups from the amino acid side chains, using the mixtureTFA/TIPS|/H₂O (92:4:4, v/v/v). The reaction was carried out for two hours on a laboratory shaker. The resin was then drained under reduced pressure on a filter funnel and the filtrate was concentrated to a volume of about 2 ml with a vacuum evaporator. The remaining filtrate was treated with diethyl ether (cooled to about 4° C) to give a white precipitate. After centrifugation in a centrifuge tube for 15 minutes (4500 x g) the supernatant was decanted and the pellet was treated with another portion of Et₂O. The precipitate was washed 3 times as described above, then dried in a vacuum desiccator. The obtained raw product was dissolved in water and freeze-dried.

### Purification:

The compound was purified using high performance liquid chromatography. The crude product of the synthesis was dissolved in water and injected into a Jupiter® Proteo C12 semipreparative column (Phenomenex) with dimensions 21.2 mm x 250 mm, 90 Å, 4 µm. The chromatographic separation was carried out in a linear gradient of 5-100% B over 180 min with eluents: A = 0.1% TFA in H₂O and B = 0.1% TFA, 25% ACN in H₂O. The eluent flow rate was 13 ml/min, UV detection at λ = 223 nm.

### Characterization of the product - HPLC and mass spectrometry:

The crude product was subjected to chromatographic analysis using high performance reverse phase liquid chromatography (RP-HPLC). [Chromatographic column: Kinetex 2.6 µm 100 Å, 100 x 2.1 mm. Eluents: A = 0.1% TFA in H₂O, B = 0.1% TFA, 80% ACN in water, flow rate 0,5 ml/min, UV detection at λ = 223 nm, linear gradient of 5% B → 100% B over 10 minutes, temperature of oven 40° C, Rₜ = 4.07 minutes]. The calculated molecular weight of the compound was confirmed with an LC MS IT-TOF (Shimadzu) device. Theoretical average molecular weight of the compound: 2767.04 Da, obtained m/z: 2767.05 ([M]⁺) (after deconvolution). [Chromatographic column Kromasil C8 5 µm 100 Å, 250 x 1.0 mm. Eluents: A = 0.2% formic acid in H₂O, B = 0.2 % formic acid, 100%, ACN in H₂O, flow rate 0.08 ml/min, detector ELSD and PDA, liner gradient of 5% B → 100% B over 25 min.]

### Exchange of trifluoroacetic counterion with acetate ion:

Exchange of the trifluoroacetic counterion with acetate ion was performed on a Strata X-C 33U Polymeric Strong Cation column according to the procedure:
- washing the column with methanol, then with a 0.1 M solution of acetic acid in water,
- dissolving the compound in water and applying to the equilibrated column,
- binding the compound to the bed of the column, with progress being monitored by RP-HPLC,
- if the compound has bound to the column, washing with 0.1 M aqueous acetic acid,
- eluting the compound with 1 M aqueous ammonium acetate in 50% MeOH (v/v).
The eluate was evaporated with a vacuum evaporator and lyophilized. The lyophilisate was redissolved in water and re-lyophilized to remove remaining ammonium acetate.

### II. BIOLOGICAL TESTS

### Example 7:

### The effect of PDGF derivatives on proliferation of skin cells (XTT test) and their cytotoxicity to skin cells(LDH test)

The effect of PDGF derivatives (PDGF1, AcPDGF1, PDGF2, PGDF3, PDGF2_NE, PDGF2-HTT) on the proliferation of immortalized human 46BR.1N skin fibroblast cells, HaCaT skin keratinocytes (for each peptide 4 experimental cycles for HaCaT and 46BR.1N cells) and primary skin fibroblasts (for each peptide 4 experimental cycles, usingcells isolated from 4 patients) was investigated. Their cytotoxicity to these cells was also checked. The cells were incubated with peptides previously dissolved in double-distilled, sterile water. Proliferation tests (XTT test) were performed at the following concentrations: 0.01, 0.1, 1, 10, 25, 50, 100 and 150 µg / ml. Cytotoxicity tests (LDH) were performed at concentrations of 50, 100 and 150 µg / ml.

### Effect of the compound PDGF1 on the proliferation of skin cells, and its cytotoxicity to these cells

PDGF1 stimulates proliferation of(statistically significant differences, U-Mann Whitney test, p <0.05):
- 46BR.1N fibroblasts, with an increase in proliferation of 20-70% versus control at concentrations of 0.1, 1, 10 µg / ml after 72h incubation,
- HaCaT keratinocytes, with an increase in proliferation of 10-50% relative to control at concentrations of 0.1 µg / ml after 48h and 0.1 and 1 µg / ml after 72h incubation,
- primary fibroblasts, with an increase in proliferation of 10-60% compared to the control at concentrations of 0.1, 1, 10, 25 and 50 µg / ml after 48h and concentrations of 1, 10, 25 and 50 µg / ml after 72h incubation.
At a concentration of 150 µg / ml it causes a slight inhibition of proliferation (10-20%) of 46BR.1N fibroblasts after 48 and 72 hours of incubation, andHaCaT keratinocytes after 72 hours of incubation at the same concentration. It does not inhibit the proliferation of primary fibroblasts. This compound is not cytotoxic to the tested cells (Fig. 7).

### Effect of the compound AcPDGF1 on the proliferation of skin cells, and its cytotoxicity to these cells:

AcPDGF1 stimulates proliferation of (statistically significant differences, U-Mann Whitney test, p <0.05):
- 46BR.1N fibroblasts, with an increase in proliferation of 20-60% versus control at concentrations of 1 and 10 µg / ml after 72h incubation,
- HaCaT keratinocytes, with an increase in proliferation of 10-70% relative to control at concentrations of 0.1, 1, 10, 25, 50 and 100 µg / ml after 48h and concentrations of 0.1, 1 and 50 µg / ml after 72h incubation\
- primary fibroblasts, with an increase in proliferation of 10-40% compared to the control at concentrations of 1, 10 and 25 µg / ml after 48h and concentrations of 1 and 10 µg / ml after 72h incubation
At a concentration of 150 µg / ml it causes a slight inhibition of 46BR.1N fibroblast proliferation (10-30%) after 72h incubation. It does not inhibit the proliferation of HaCaT keratinocytes or primary fibroblasts. This compound is not cytotoxic to the tested cells (Fig. 8).

### Effect of the compound PDGF2 on the proliferation of skin cells, and its cytotoxicity to these cells:

PDGF2 stimulates proliferation of(statistically significant differences, U-Mann Whitney test, p <0.05).:
- 46BR.1N fibroblasts, with an increase in proliferation of 20-90% relative to the control at concentrations of 0.01, 0.1, 1, 10, 25 and 50 µg / ml after 48h and concentrations of 0.1, 1 and 10 µg / ml after 72h incubation,
- HaCaT keratinocytes, with an increase in proliferation of 20-140% relative to the control in concentrations of 0.1, 1, 10, 25 and 50 µg / ml after 48h incubation and concentrations of 0.1, 1, 10, 25, 50, 100 and 150 µg / ml after 72h incubation,
- primary fibroblasts, with an increase in proliferation of 10-40% relative to control at concentrations of 0.1, 1, 10, 25 and 50 µg / ml after 48h and concentrations of 1, 10 and 25µg / ml after 72h incubation
PDGF2 does not inhibit the proliferation of the tested cells and is not cytotoxic to them (Fig. 9).

### Effect of the compound PDGF2 HTT on the proliferation of skin cells, and its cytotoxicity to these cells:

PDGF2-HTT stimulates proliferation of:
- 46BR.1N fibroblasts, with an increase in proliferation of 10-40% relative to controlin concentrations of 0.1 µg / ml after 48h and 1 µg / ml after 72h incubation,
- HaCaT keratinocytes, with an increase in proliferation of 10-60% relative to control in concentrations of 0.1, 1, 10 and 25 µg / ml after 48h incubation and concentrations of 0.1, 1, 10 and 25 µg / ml after 72h incubation,
- primary fibroblasts, with an increase in proliferation of 10-60% relative to control at concentrations of 1, 10 and 25 µg / ml after 48h and concentrations of 0.1, 1, 10 and 25 µg / ml after 72h incubation (statistically significant differences, U-Mann Whitney test, p <0.05).
At certain concentrations PDGF2-HTT inhibits skin cell proliferation. With 46BR.1N fibroblasts it reduces proliferation by 20-70% relative to control at concentrations of 50, 100 and 150 µg / ml after 48 and 72h, with HaCaT keratinocytes it reduces proliferation by 40-80% relative to control in concentrations of 100 and 150 µg / ml after 48 and 72h, and with primary fibroblasts it reduces proliferation by 10-70% (depending on the patient) compared to the control at concentrations of 100 and 150 µg / ml (statistically significant differences, U-Mann Whitney test, p <0.05). In addition, it is cytotoxic to 46BR.1N fibroblasts at concentrations of 50, 100 and 150 µg / ml, toHaCaT keratinocytes at concentrations of 100 and 150 µg / ml and to primary fibroblasts at a concentration of 150 µg / ml (statistically significant differences, U-Mann Whitney test, p <0.05) (Fig. 10).

### Effect of the compound PDGF2-NE on the proliferation of skin cells, and its cytotoxicity to these cells:

PDGF2_NE stimulates proliferation of(statistically significant differences, U-Mann Whitney test, p <0.05):
- 46BR.1N fibroblasts, with an increase in proliferation of 20-40% relative to control in concentrations of 1; and 10 µg / ml after 48h and concentrations of 1, 10, 25 and 50 µg / ml after 72h incubation,
- HaCaT keratinocytes, with an increase in proliferation of 20-60% relative to control by in concentrations of 0.1, 1, 10 and 25 µg / ml after 48h incubation and concentrations of 0.1, 1, 10, 25, 50, 100 and 150 µg / ml after 72h incubation,
- primary fibroblasts, with an increase in proliferation of 10-40% compared to control inconcentrations of 1, 10 and 25 µg / ml after 48h and concentrations of 0.1, 1, 10, 25 and 50 µg / ml after 72h incubation.
PDGF2_NE does not inhibit the proliferation of tested cells and is not cytotoxic to them (Fig. 11).

### Effect of the compound PDGF3 on the proliferation of skin cells, and its cytotoxicity to these cells:

PDGF3 stimulates proliferation of(statistically significant differences, U-Mann Whitney test, p < 0.05).:
- 46BR.1N fibroblasts, with an increase in proliferation of 20-40% relative to control in concentrations of 0.1, 1 and 10 µg / ml after 48h and 72h incubation,
- HaCaT keratinocytes, with an increase in proliferation of 20-120% relative to control in concentrations of 0.1, 1, 10 and 25 µg / ml after 48h incubation and concentrations of 0.1, 1, 10, 25, 50, 100 and 150 µg / ml after 72h incubation,
- and primary fibroblasts, with an increase in proliferation of 10-60% relative to control at concentrations of 1, 10, 25 and 50 µg / ml after 48h and 72h incubation
At a concentration of 150 µg / ml, it causes a slight inhibition of proliferation (10-20%) of 46BR.1N fibroblasts after 72 hours of incubation. It does not inhibit the proliferation of HaCaT keratinocytes and primary fibroblasts. This compound is not cytotoxic to the tested cells (Fig. 12).

### Example 8:

### Effect of the compound PDGF2 on chemotaxis of HaCaT keratinocytes and the effect of compounds PDGF2 and PDGF3 on primary fibroblast chemotaxis

The effect of the compound PDGF2 compound at a concentration of 1 µg / ml on the chemotaxis of HaCaT keratinocytes, and the effect of PDGF2 and PDGF3 at a concentration of 1 µg / ml on the chemotaxis of primary fibroblasts, were investigated. Cells were "starved" in serum-free medium for 24 hours before the experiment. They were then seeded in serum-free medium in culture inserts (Grainer bio one, 8 µm) placed in a 24-well plate. Serum-free medium containing PDGF2 or PDGF3 was added to the wells. Controls in the test were: serum free medium and medium containing 10% FBS. After 24 hours of incubation, the cells were stained with calcein. Cells that migrated through the insert membrane were then detached with trypsin and a fluorometric measurement was performed (excitation wavelength 485, emission wavelength 520 nm).

A slight chemotactic effect of the compound PDGF2 on the examined cells was observed (10-30% compared to controls) (Fig. 13 top). Results obtained for fibroblasts show that the compounds PDGF2 and PDGF3, at a concentration of 1 µg / ml, inhibit the chemotaxis of human primary dermal fibroblasts (Fig. 13 bottom).

### Example 9:

### PDGF2 peptide stability in human plasma

Blood was collected from healthy individuals and centrifuged in lithium/heparin tubes. EDTA was then used as an anticoagulant. The plasma was aliquoted and incubated with PDGF2 for 1, 2, 3, 6 and 24 h at 37°C, at a final concentration of 144 µM. The samples were prepared in sterile conditions. After the appropriate time, a 4-fold excess of absolute ethanol (v/v) was added, the mixture was kept on ice for 15 min and then centrifuged (32000 rcf, 4°C, 20 min). The supernatant was collected, air-dried under vacuum and dissolved in 100 µL of 0.01% TFA in H₂O. The analysis was carried out by HPLC equipped with a Phenomenex Luna C18(2) (5 µm, 100 Å, 4.6 x 250 mm) column and monitored by PDA. 0.01% TFA in H₂O (A) and 0.01% TFA in 80% MeCN in water (B) were used as solvents. A linear gradient of 5-100% over 60 min and a flow rate of 1 mL/min was applied. For calculations, peak areas were compared to a calibration curve calculated by Shimadzu LCsolution Software.

After 24 hour incubation at 37 °C, the signal of PDGF2 peptide is no longer seen (Fig. 14).

### Example 10 :

### Study of the interactions between PDGF-2 peptide and bovine albumin

### Preparation of microcolumn for affinity chromatography:

The following buffers were used to prepare the column and carry out affinity tests:
1. *coupling buffer, CB* - 0.2M NaHCO₃ and 0.5M NaCl (pH 8.3),
2. *phosphate buffer saline, PBS* - 5mM Na₂HPO₄ and 150 mMNaCl (pH 7.4),
3. *blocking buffer, BB* - 0.1 M ethanolamine and 0,5 M NaCl (pH 8.3),
4. *washing buffer, WB* - 0.2 M CH₃COONa and 0,5 M NaCl (pH 4),
5. *ammonium hydrogen carbonate* - 50 mM NH₄HCO₃ (pH 7.4).

In order to perform the test for affinity of the PDGF-2 peptide to the albumin, an Abo Mobicol "F" microsphere (# M105035F) was used. 100 µg of albumin protein was weighed and dissolved in 100 µl of 0.2 mM coupling buffer. 0.06 g of sepharose (Activated CH-Sepharose 4B, A9019, Sigma-Aldrich) was weighed into the microcolumn and soaked in coupling buffer for 5 minutes for the resin to swell. The dissolved albumin was transferred to the microcolumn and incubated for two hours at 25 °C, with gentle shaking. The microcolumn was washed with the blocking and washing buffers, four times for each. The column was again incubated for 1 hour, with gentle shaking at 25 °C, in the blocking buffer. After incubation the column was again washed with the blocking and washing buffers, four times for each.

### The affinity test procedure:

The albumin-bound affinity column was equilibrated with 20 ml of 50 mM ammonium hydrogen carbonate. 20 µg of peptide, dissolved in 20 µl of ammonium hydrogen carbonate, was then added and incubated at 25 °C for 2 hours with gentle shaking. After 2 hours, the column was washed with 100 ml of ammonium hydrogen carbonate, collecting the first and last milliliter of the solution (supernatant and last wash fraction). The albumin-peptide complex was dissociated by incubation for 15 minutes in 0.1% TFA, with gentle shaking. The procedure was repeated twice and the elution fraction was collected each time. The microcolumn was washed with PBS solution to renature the immobilized protein and stored at 4 °C. All fractions were lyophilised and analyzed by mass spectrometry (SHIMADZU ESI-IT-TOF).

### Analysis of the mass spectra:

The mass spectrum of the last wash fraction indicates that the column was properly washed out. (FIG. 15, left) No signal was observed, confirming that the excess of PDGF-2 peptide had been removed. The spectrum of the elution fraction shows the peak m/z 1331.746 (Fig. 15, right), which is consistent with the mass of the PDGF-2 peptide.

It can be concluded that the PDGF-2 peptide interacts with bovine albumin, since the peak m/z in elution fraction is consistent with the mass of the peptide.

### Example 11:

### Study of the effect of the compound PDGF2 on the expression levels of pluripotency genes and genes regulating the cell cycle of the keratinocyte cell line cultures

To determine the effect of PDGF derivatives on the expression levels of pluripotency genes and genes regulating the cell cycle, a number of human keratinocyte cell line cultures taken from patients were isolated. The cultures were stimulated with the PDGF derivative PDGF2 at 3 different concentrations (0.1 µg/ml, 1.0 µg/ml and 10 µg/ml) and one was stimulated with 5% FBS. As a control, a culture of the cell line, without the addition of FBS, was chosen. Transcript levels for the gene panel including *CDKN1A, MYC, KIT, POU5F1, TGFB3, TP53* were examined. RNA isolation was performed using a RNeasy kit (Qiagen). Levels of selected transcripts were determined using real-time quantitative PCR using *TBP* and *ACTB* as reference transcripts. The results (Fig. 16) do not show statistically significant changes in the level of the selected transcripts in the presence of the tested compound.

The lack of transcriptional activation of the analyzed regulatory factors, including those related to the oncogenesis of *MYC* and *KIT,* indicates that the PDGF2 peptide tested does not interfere with the functioning of keratinocytes.

### Example 12:

### Analysis of the immunogenic properties of PDGF2

Tests were conducted on human peripheral blood mononuclear cells (PBMC), isolated from "buffy coats" using a ficoll density gradient (histopaque, Sigma). Following two wash steps (in PBS), erythrocyte lysis and cell counting (cell counter, Bio-Rad), the PBMCs were seeded onto a 24-well culture plate at a density of 1 mln cells/1 ml of RPMI 1640 (P/S, 10% FBS)/well. Thecells were then allowed to adapt to the culture conditions for the next 24h. After cell resting, PDGF2 was added to the wells in a final concentration of 1.0 µg/ml, and the cells were incubated for the next 48h under appropriate conditions (37°C, 5% CO₂). The untreated cells constituted a negative control, whereas cells stimulated with LPS (1 µg/ml) and PHA (2,5 µg/ml) were a positive control. After incubation, the PBMCs were collected, washed with PBS, counted and prepared for flow cytometry analysis under the following conditions: 10⁴ cells/ 100µl were stained with monoclonal, fluorochrome-stained antibodies (anti-CD3, CD4, CD8, CD16, CD56, CD25, CD69, CD71, HLA-DR, CD11c, CD80, CD83). Following 30 min of incubation at RT in the dark, the cells were analyzed using a flow cytometer (LSRFortessa, BD).

### Immunogenicity and immunological safety of PDGF2

An analysis of peptide impact on the activity of immune cells was performed on human PBMCs. These cells were incubated with PDGF2 (1.0 ug/ml) for 48h and the effects of stimulation were evaluated by flow cytometry. We assessed the activation level of T cells (CD3/CD4/CD8) and NK cells (CD16/CD56) through the evaluation of expression of the activation markers CD69, CD71, CD25 and HLA-DR. The influence of the examined compound on dendritic cells (CD11c, HLA-DR) was also investigated, by assessment of the expression of CD80 and CD83 molecules.

The obtained results show no immunogenic properties of PDGF2. The observed activation levels of the analyzed immune cells (CTL, Th, NK) were comparable to negative control levels (Fig. 17).

Similar observations (Fig. 18) were made for particular cell subpopulations. Moreover, a statistically significant decrease in activity was noted for cells stimulated with PDGF2 (p<0,05) compared to the positive control (LPS/PHA). In addition, dendritic cells were not activated in presence of PDGF2 (Fig.19) (inactive phenotype of DC - CD11c+ CD80-, CD83-; Active phenotype - CD11+, CD80+, CD83+) Therefore, based on the conducted test, the immunological safety of PDGF2 was confirmed.

### Example 13:

### Basophil activation test (BAT) - evaluation of the allergenic potential of PDGF2

The commercially available test Flow CAST® highsens (Bühlmann Laboratories, Switzerland) was used to assess the activity of granulocytes. The test was performed on blood samples collected from healthy volunteers, within 24h of collection, according to the protocol. 100µl of blood was incubated with PDGF2 at a final concentration of 1.0 µg/ml. The cells were then stained with fluorochrome-stained monoclonal antibodies (anti-CD63, CD203c, CCR3) and incubated for 15 min at 37°C. After incubation, erythrocytes were lysed and the cells were washed, then subjected to flow cytometry analysis (BD FACSCanto II). Each sample was accompanied by its own negative and positive control (anti-FcεRI mAb andfMLP). The obtained results (Fig.20) show no granulocyte activation in the presence of PDGF2 (1.0 µg/ml), as they show values comparable to negative control.

### Example 14:

### Assessment of transcriptomic profile of ASCs and fibroblasts under the influence of the PDGF2 peptide

The influence of PDGF2 on the transcriptomic profile of primary cell cultures of adipose stem cells and skin fibroblasts was studied. Human subcutaneous adipose tissue was sampled from three patients of the Plastic Surgery Clinic of the Medical University of Gdansk. The procedure was approved by the Independent Bioethics Commission for Research of the Medical University of Gdansk (NKBBN/387/2014). The isolation of adipose-derived stem cells (ASCs) was based on a standard protocol: following enzymatic digestion and erythrocyte lysis, the cells were suspended in the culture medium Dulbecco's modified Eagle medium (DMEM low glucose - 1000 mg) supplemented with 10% fetal bovine serum FBS. The isolation of fibroblasts was performed according to the protocol described by Kosikowska&Pikula (2015), which utilizes collagenase enzyme followed by culturing in DMEM medium with high glucose content. After the 2nd passage (P2) the cells were seeded and cultured for 24h in media supplemented with 10% FBS, followed by culturing for 24h with 5% FBS, and then culturing for 48h in media without FBS but supplemented with PDGF2 peptide (1 ug/ml). ASC cultures deprived of FBS for 48h were used as a control. After the stimulation the cells were trypsynized, collected, centrifuged and the pellet was snap frozen for transcriptomic analysis. RNA isolated from three replicates with RNA Integrity Number (RIN) above 7.0 were pooled in equal amounts (by mass), and later used for massive parallel transcriptome sequencing. The readings were mapped with reference to the human genome (hgl9 edition) using TopHat software. Differential expression analyses between the FBS-deprived condition and the condition with added PDGF2 were made with the Cufflinks package, using the complete workflow for version 2.2.0 (and higher), as described by the authors (http://cole-trapnell-lab.github .io/cufflinks/manual/). The results of comparative analyses were used to build DGE (Differential Gene Expression) matrices that were further processed by Ingenuity Pathway Analysis (IPA).

IPA enables analysis of transcript changes in experimental datasets and assigning them to shifts in upstream regulators and downstream effects. The consistency score is a measurement used to describe this relationship. The regulatory network with the highest consistency score (6,414) for ASCs revealed distinct activation of cell cycle progression and inhibition of lymphocyte T migration (Fig. 21). The analysis with the same settings for fibroblasts stimulated with PDGF2 gave none-regulatory networks. Additionally, the activation of effects related to cell cycle progression and cell viability, with concurrent inhibition of inflammatory responses, was observed for ASCs cultured with PDGF2 (Table 1). Similar effects related to cell and stem cell migration were found for fibroblasts stimulated with that peptide (Table 2).

**Table 1: Diseases and functions predicted to be influenced in ASCs originating from three patients after RNA pooling and stimulation with PDGF2 (1 µg/ml). Z-score is an algorithm in IPA designed to reduce the chance that random data will produce a significant prediction. It identifies functions with the strongest prediction for increase (positive z-score) or decrease (negative z-score). Values of p-value <0.05 and z-score ≤-2 or ≥2 are considered significant.**

| **Diseases and functionannotation** | **z-score** | **Predicted activationstate** | **p-value** | **Molecules** |
|---|---|---|---|---|
| **Invasion of cells** | 7.01E-08 | Increased | 2.520 | 77 |
| **Cell cycleprogression** | 1.55E-05 | Increased | 2.498 | 62 |
| **Proliferation of epithelial cell lines** | 1.84E-05 | Increased | 2.336 | 30 |
| **Binding of professional phagocytic cells** | 8.97E-07 | Decreased | -2.001 | 22 |
| **Flux of cation** | 1.19E-06 | Decreased | -2.037 | 26 |
| **Cell viability of lymphocytes** | 1.61E-04 | Decreased | -2.051 | 10 |
| **Cell movement of lymphocytes** | 5.72E-07 | Decreased | -2.103 | 30 |
| **Cell movement of phagocytes** | 1.51E-04 | Decreased | -2.104 | 31 |
| **Binding of bloodcells** | 2.60E-12 | Decreased | -2.107 | 49 |
| **Migration of mononuclearleukocytes** | 7.85E-09 | Decreased | -2.118 | 35 |
| **Cell death of cancer cells** | 1. 13E-04 | Decreased | -2.128 | 26 |
| **Lymphocytemigration** | 1.38E-07 | Decreased | -2.141 | 29 |
| **Lymphopoiesis** | 1.08E-05 | Decreased | -2.151 | 22 |
| **Flux of ion** | 2.17E-07 | Decreased | -2.164 | 28 |
| **Cellular homeostasis** | 1.51E-05 | Decreased | -2.214 | 82 |
| **Flux of Ca²⁺** | 1.25E-05 | Decreased | -2.342 | 23 |
| **Lymphohematopoieticneoplasia** | 1.49E-14 | Decreased | -2.345 | 319 |
| **Hematologicalneoplasia** | 3.76E-14 | Decreased | -2.345 | 319 |
| **Cell movement of mononuclear leukocytes** | 7.26E-07 | Decreased | -2.485 | 38 |
| **Cell movement of T lymphocytes** | 4.67E-05 | Decreased | -2.486 | 18 |
| **T cellmigration** | 6.95E-05 | Decreased | -2.522 | 19 |
| **Binding of mononuclearleukocytes** | 1.08E-07 | Decreased | -2.563 | 25 |
| **Mobilization of Ca2+** | 1.96E-06 | Decreased | -2.944 | 30 |

**Table 2: Diseases and functions predicted to be influenced in fibroblasts originating from three patients after RNA pooling and stimulation with PDGF2 (1 µg/ml). Z-score is an algorithm in IPA designed to reduce the chance that random data will produce significant prediction. It identifies functions with the strongest prediction for increase (positive z-score) or decrease (negative z-score). Values of p-value <0.05 and z- score ≤-2 or ≥2 are considered significant.**

| **Diseases and functionannotation** | **z-score** | **Predictedactivationstate** | **p-value** | **Molecules** |
|---|---|---|---|---|
| **Cell movements** | 2.83E-03 | Increased | 3.742 | 76 |
| **Cell migration** | 1.53E-03 | Increased | 3.445 | 70 |
| **L-tyrosinephosphorylation** | 3.11E-02 | Increased | 2.449 | 8 |
| **Stemcellmigration** | 4.20E-04 | Increased | 2.176 | 5 |
| **Invasion of cells** | 1.36E-02 | Increased | 2.073 | 40 |
| **Apoptosis** | 1.57E-03 | Decreased | -2.016 | 88 |

### III. ANIMAL MODEL

### Example 15:

### Effect of topical administration of PDGF2 compound on the healing of skin wounds

The effect of topical administration of the compound PDGF2 on skin wound healing in mice was determined using a model of dorsal skin injury. Females (8 weeks old) of the Balb/C laboratory mouse strain were used for the experiments. Before the damage was inflicted, the mice were subjected to general anesthesia, and the dorsal skin was shaved and disinfected. Two symmetrical holes were made in the skin on the back using a biopsy punch with a diameter of 6 mm. Immediately after wounding, the compound PDFG2 in a hydrogel (P407) (25 µg/ml) was applied as one drop of 25 µl directly to the wound and then once a day for 4 consecutive days. After each administration of PDGF2, after solidification of the hydrogel, the wounds were covered with a transparent Tegaderm dressing, which was additionally fastened with a adhesive plaster wrapped around the torso of the mouse. The experiment was carried with a group of 6 mice, and an analogous procedure was performed for the control group, which was administered only the P407 hydrogel. In the first week of the experiment, the P407 hydrogel or P407 hydrogel with PDGF2 peptide was administered once daily for 5 days, including the date of injury. In the second week the dressing was changed every other day. The dressing was removed at the beginning of the third week. After the third week, the animals were euthanized. Dorsal skin was collected, spread on a cork and placed in formalin. Wounds were photographed on the day of damage (d0) and 2, 4, 7, 11, 14, and 18 days after wounding. The healing progress was measured by determining the surface of the wound using computer image analysis. The protocol of experiments on animals was approved by the Local Ethical Committee for Animal Experiments at the University of Technology and Life Sciences in Bydgoszcz (permission no. 49/2016).

### Skin wound healing:

During the first week of healing, we did not observe a drastic reduction of the wound surface (Fig. 22 and Fig. 23). At the end of the first week, we observed a slight reduction of the wound area and the onset of epidermis. Although in mice treated with the hydrogel with the PDGF2 peptide, the mean wound area changes were similar to those of control mice treated with P407 hydrogel alone, in tissues treated with the PDGF2 peptide this process is slightly more intense. On day 11 after the administration of PDGF2, an acceleration of epidermal formation compared to that of control was observed. By day 14the wounds in both groups were completely closed and a small scar was created. In the third week, the scar was slightly reduced in both groups, however in the PDGF2 peptide groups these scars fade very quickly and are less visible (Fig. 22).

### Histological imaging:

The skin was fixed in formalin for a week, after which it was embedded in paraffin and cut into 5 µm sections. The samples were stained with hematoxylin and eosin or with Masson's trichrome staining. The samples were analyzed under a light microscope.

The control group showed extensive scar tissue with highly marked collagen (Fig. 24). Inside the scar there was noticeable sporadic formation of hair follicles, however no gland formation was observed. The outlets of the hair follicles could be seen on the edges of the scar. In the samples from the group receiving PDGF2, scar tissue could also be seen, however it is less extensive compared to the control. The collagen structure was looser and less regular, especially in the deeper scar regions. We also observed much more frequent occurrences of hair follicles and glands. Those skilled in the art can also observe cell areas differing in morphology from the surrounding fibroblasts.

### Example 16:

### Pharmaceutical composition:

Pharmaceutical compositions comprise at least one of the new compounds (PGDF1; Ac-PGDF1; PGDF2; PGDF2-HTT, PGDF2-NE1; PGDF3) and pharmaceutically acceptable carriers and/or diluents.

"Pharmaceutically acceptable" means a diluent, buffer, carrier or excipient which, at the dosage and concentrations employed, does not cause any unwanted effects in the patients to whom it is administered. Such pharmaceutically acceptable buffers, carriers or excipients are well-known in the art(see Remington's Pharmaceutical Sciences 18th edition, A.T Gennaro, Ed., Mack Publishing Company (1990) and the Handbook of Pharmaceutical Excipients, 3rd edition, A.Kibbe, Ed., Pharmaceutical Press (2000)).

The pharmaceutical composition may be admixed with additives (adjuvants) such as lactose, sucrose, starch powder, cellulose esters of alkanoic acids, stearic acid, talc, magnesium stearate, sodium and magnesium salts of phosphoric and sulfuric acids, acacia, gelatin, sodium alginate, polyvinyl-pyrrolidine and/or polyvinyl alcohol, and tableted or encapsulated for conventional administration.

Alternatively, they may be dissolved in saline, water, polyethylene glycol, propylene glycol, ethanol, ethanol, oils, tragacanth gum and/or various buffers. Other adjuvants and modes of administration are well known in the pharmaceutical art. The carrier and diluent may include time-delay material, such as glycerol monostearate or glycerol distearate, alone or with wax or other materials well known in the art.

The pharmaceutical composition may be subjected to conventional pharmaceutical operations, such as sterilization and/or may contain conventional adjuvants such as preservatives, stabilisers, wetting agents, emulsifiers, buffers, fillers, etc., e.g. as disclosed elsewhere herein.

The solid and liquid forms of the pharmaceutical compositions are, for example, granules, powders, tablets, coated tablets, (micro)capsules, suppositories, syrups, emulsions, gels, ointments, suspensions, creams, aerosols, drops or injectable solutions in ampule form, and also preparations for protracted release of active compound, in whose preparation excipients, diluents, adjuvants or carriers are customarily used as described above. The pharmaceutical composition may also be provided in bandages or plasters or the like.

The pharmaceutical composition will be administered to a patient in pharmaceutically effective doses. By "pharmaceutically effective dose" is meant a dose that is sufficient to produce the desired effects in relation to the condition for which it is administered. The exact dose is dependent on the activity of the compound, manner of administration, nature and severity of the disorder, age and body weight of the patient, and adjustment of dosage may thus be needed. The administration of the dose can be carried out in the form of an individual dose unit or several smaller dose units, and may involve multiple administration of subdivided doses at specific intervals. The pharmaceutical composition of the invention may be administered aloneor in combination with other therapeutic agents, such as antibiotics or antiseptic gents, for example penicillinsor cephalosporins.

### Conclusions:

PDGF2 used on wounds causes a slight acceleration of the healing process, however, it should be remembered that mouse skin heals very quickly and thus the quality of the reconstructed tissue is much more important in this model. In tissues stimulated with the PDGF2 peptide the process of filling the wound with tissue begins earlier than in the control. The use of PDGF2 peptide significantly affects the quality of the reconstituted tissue. The resulting tissue does not show classic scar morphology (Fig. 24). The collagen in the tissue is arranged in a less orderly manner, which is characteristic of normal skin. This is important because tightly packed parallel collagen fibers are much less resistant to mechanical stresses. The more frequent occurrence of skin attributes in the reconstructed region also indicates increased healing effectiveness. It is also worth noting that the new tissue, after the initial healing process, is subject to transformations and maturation lasting from a week to several months. During this process, changes take place in the tissue to increase the strength and reduce the excess of collagen formed during healing. Observed areas with altered morphology suggest the occurrence of differentiation processes, which may lead to elements present in normal skin, such as hair follicles, glands, fat tissue or muscle. This allows us to suppose that PDGF2 positively affects the maturation of tissue formed in the healing process.

In the first week after wounding, we observed the formation of membranes in the wound. It is noted that the these "membranes" (partially differentiated epidermis) in the PDGF2 group appear faster, are thicker and mechanically stronger (Fig. 25) than in the control. Analysis of histological preparations showed significant differences in the thickness of the resulting tissue. Membranes from the test group also show a higher cell density compared to the control (Fig. 26).

### Literature:

[1] Piku 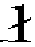 a M., Langa P., Kosikowska P., Trzonkowski P.: Komórki macierzyste i czynniki wzrostu w gojeniu ran; PostepyHigMedDosw, 2015; 69: 874-885
[2] Kaltalioglu K., Coskun-Cevher S., Tugcu-Demiroz F., Celebi N.: PDGF supplementation alters oxidative events in wound healing process: a time course study; Arch Dermatol Res, 2013; 305:415-422
[3] Cheng B., Liu HW., Fu XB., Sun TZ., Sheng ZY.: Recombinant human platelet-derived growth factor enhanced dermal wound healing by a pathway involving ERK and c-fos in diabetic rats; J Dermatol Sci., 2007; 45(3):193-201
[4] Li H., Fu X., Zhang L., Huang Q., Wu Z., Sun T.: Research of PDGF-BB gel on the wound healing of diabetic rats and its pharmacodynamics; J Surg Res. 2008 Mar;145(1):41-8.
[5] Werner S., Grose R.: Regulation of Wound Healing by Growth Factors and Cytokines; Physiol Rev. 2003 Jul;83(3):835-70
[6] Barrientos S., Brem H, Stojadinovic O, Tomic-Canic M.: Clinical application of growth factors and cytokines in wound healing; Wound Repair Regen. 2014 Sep-Oct;22(5):569-78.
[7] Barrientos S., Stojadinovic O., Golinko M.S., Brem H., Tomic-Canic M.: Growth factors and cytokines in wound healing.; Wound Repair Regen. 2008 Sep-Oct;16(5):585-601
[8] Shah P., Keppler L., Rutkowski J.: A Review of Platelet Derived Growth Factor Playing Pivotal Role in Bone Regeneration;J Oral Implantol. 2014 Jun;40(3):330-40
[9] Dreifke MB, Jayasuriya AA, Jayasuriya AC. Current wound healing procedures and potential care. Materials science & engineering C, Materials for biological applications. 2015;48:651-662.
[10] Bowen-Pope DF., Raines EW.: History of Discovery: Platelet-derived Growth Factor; ArteriosclerThrombVasc Biol. 2011 November ; 31(11): 2397-2401
[11]Das S., Majid M., Baker A.B: Syndecan-4 enhances PDGF-BB activity in diabetic wound healing; Acta Biomater. 2016 Sep 15;42:56-65
[12] Gieringer M, Gosepath J, Naim R. Radiotherapy and wound healing: principles, management and prospects (review). Oncol Rep. 2011 Aug;26(2):299-307
[13] Falcone RE, Nappi JF. Chemotherapy and wound healing. SurgClin North Am. 1984 Aug;64(4):779-94.
[14] McCaw DL. The effects of cancer and cancer therapies on wound healing. Semin Vet Med Surg (Small Anim). 1989 Nov;4(4):281-6.
[15] Rybinski B, Franco-Barraza J, Cukierman E. The wound healing, chronic fibrosis, and cancer progression triad. Physiol Genomics. 2014 Apr 1;46(7):223-44.
[16] Brem H, Tomic-Canic M, Tarnovskaya A, Ehrlich HP, Baskin-Bey E, Gill K, Carasa M, Weinberger S, Entero H, Vladeck B. Healing of elderly patients with diabetic foot ulcers, venous stasis ulcers, and pressure ulcers. Surg Technol Int. 2003; 11:161-7.
[17] Blakytny R, Jude E.The molecular biology of chronic wounds and delayed healing in diabetes. Diabet Med. 2006 Jun;23(6):594-608.
[18] Deptu 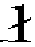 a M, Wardowska A, Dzierżyńska M, Rodziewicz-Motowid 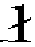 o S, Piku 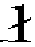 a M. Antibacterial Peptides in Dermatology-Strategies for Evaluation of Allergic Potential. Molecules. 2018 Feb 14;23(2).
[19] Xiao Y, Lewis A, Reis LA, Feric N, Knee EJ, Gu J, Cao S, Laschinger C, Londono C, Antolovich J, McGuigan AP, Radisic M. Diabetic wound regeneration using peptide-modified hydrogels to target re-epithelialization. Proc Natl Acad Sci USA. 2016; 113(40): E5792-E5801.
[20]Eming SA, Martin P, Tomic-Canic M. Wound repair and regeneration: mechanisms, signaling, and translation, Sci. Transl. Med. 6 (2014) 265sr6-265sr6.
[21] Lindblad WJ. Considerations for selecting the correct animal model for dermal wound-healing studies, J. Biomater. Sci. Polym. Ed. 19 (2008) 1087- 1096.
[22] Wong VW, Sorkin M, Glotzbach JP, Longaker MT, Gurtner GC, Wong VW, Sorkin M, Glotzbach JP, Longaker MT, Gurtner GC, Surgical approaches to create murine models of human wound healing, J. Biomed. Biotechnol. 2011 (2011) 1-8.
[23]Handbook, "Affinity Chromatography: Principles and Methods", materialyfirmyAmersham Pharmacia Biotech, 2012
[23] Jaszczo 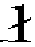 t M., Boczkaj G., Grygoryshyn K., Kaminski M., "Immunochromatografia: przegl 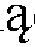 d i charakterystyka immunosorbentów oraz metod immobilizacji", Post 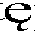 py Chromatografii, 3, 69-85, 2011.
[24] Roldan R. J., "Monoclonal antibody purification using Affinity Chromatography", Industrial Biotechnology, University of Puerto Rico at Mayaguez, 2009
[25] Zaj 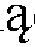 c, M.; Pawe 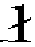 czyk, E.; Jelinska, A. Chemia leków. Poznań: Wydawnictwo Naukowe Akademii Medycznej im. Karola Marcinkowskiego; 2006. s. 90.
[26] Bajaj, B.; Singla, D.; Sakhuja, D. Stability Testing of Pharmaceutical Products, Journal of Applied Pharmaceutical Science; 2012, 02 (03), 129-138.
[27] Snape, T. J.; Astles, A. M.; Davies, J. Understanding the chemical basis of drug stability and degradation, The Pharmaceutical Journal, 2010, 285.
[28] Hartman, D. A. Determination of the stability of drugs in plasma, CurrProtocPharmacol., 2003, 7 (7.6*)*.
[29]Borthwick, A.D.; Exall, A.M.; Haley, T.M.; Jackson, D.L.; Mason, A.M.; Weingarten, G.G.; Pyrrolidine-5,5-trans-lactams as novel mechanism-based inhibitors of human cytomegalovirus protease. Part 3: potency and plasma stability, Bioorg. Med. Chem. Lett. 2002, 12, 1719-1722.
[30] Breitenlechner, C.B et al. Structure-based optimization of novel azepane derivatives as PKB inhibitors, J. Med. Chem., 2004, 47, 1375-1390.
[31] Qiu, F.; Norwood, D.L. Identification ofpharmaceuticalimpurities, J. Liq.Chromatogr.Relat.Technol., 2007, 30, 877-935.
[32] Nourah, A.; Sultan, A. et al. (). Validated Stability-Indicating Capillary Electrophoresis Method for the Separation and Determination of a Fixed-Dose Combination of Carvedilol and Hydrochlorothiazide in Tablets. J AOAC Int., 2013, 951-9.
[33] Siddiqui, M. R.; AlOthman, Z. A.; Rahman, N. Analytical techniques in pharmaceutical analysis: A review, Arabian Journal of Chemistry, 2017, 10, S1409-S1421.
[34] Kratz, F.; Elsadek, B. J Control Release,, 2012,,161: 429-445.
[35] Neumann, E.; Frei, E.; Funk, D.; Becker, M. D.; Schrenk, H. H. et al. Expert Opin Drug Deliv, 2010, 7,915-925.
[36] Kratz, F.; Beyer, U. Drug Delivery, 1998,, 5, 1-19.
[37] Naveen, R.; Akshata, K.; Pimple, S.; Chaudhari, P. Der PharmaciaSinica, 2016, 7(1), 11-15.
[38] Grabowski, T. Biofarmacja i farmakokinetyka, www.biokinetica.pl, 2007, ISBN 9788392517108.
[39] Kosikowska, P.; Pikula, M.; Langa, P.; Trzonkowski P., Obuchowski, M.; Lesner, A.: Synthesis and evaluation of biological activity of antimicrobial - pro-proliferative peptide conjugates. PLoS ONE, 2015; 10, 10, ID e140377.
[40] Gerlach, J. C. et al.Adipogenesis of Human Adipose-Derived Stem Cells Within Three-Dimensional Hollow Fiber-Based Bioreactors. Tissue Eng. Part C Methods18,54-61 (2012).
[41] Kim, D. et al.TopHat2: accurate alignment of transcriptomes in the presence of insertions, deletions and gene fusions. Genome Biol. 14,R36 (2013).
[42] Trapnell, C. et al.Transcript assembly and quantification by RNA-Seq reveals unannotated transcripts and isoform switching during cell differentiation. Nat. Biotechnol.28,511-515 (2010).
[43] Nakagami, H.; Nishikawa, T.; Tamura, N.; Maeda, A.; Hibino, H.; Mochizuki, M.; Shimosato, T.; Moriya, T.; Morishita, R.; Tamai, K.; et al. Modification of a novel angiogenic peptide, ag30, for the development of novel therapeutic agents. J. Cell. Mol. Med. 2012, 16, 1629-1639.
[44] Liu, H.; Mu, L.; Tang, J.; Shen, C.; Gao, C.; Rong, M.; Zhang, Z.; Liu, J.; Wu, X.; Yu, H.; et al. A potential wound healing-promoting peptide from frog skin. Int. J. Biochem. Cell Biol. 2014, 49, 32-41.
[45] Liu, H.; Duan, Z.; Tang, J.; Lv, Q.; Rong, M.; Lai, R. A short peptide from frog skin accelerates diabetic wound healing. FEBS J. 2014, 281, 4633-4643.
[46] Song, D.W.; Kim, S.H.; Kim, H.H.; Lee, K.H.; Ki, C.S.; Park, Y.H. Multi-biofunction of antimicrobial peptide-immobilized silk fibroin nanofiber membrane: Implications for wound healing. Acta Biomater. 2016, 39, 146-155.
[47] Di Grazia, A.; Cappiello, F.; Imanishi, A.; Mastrofrancesco, A.; Picardo, M.; Paus, R.; Mangoni, M.L. The frog skin-derived antimicrobial peptide esculentin-1a(1-21) NH2 promotes the migration of human hacat keratinocytes in an egf receptor-dependent manner: A novel promoter of human skin wound healing? PLoS ONE 2015, 10.
[48] Oudhoff, M.J.; Bolscher, J.G.; Nazmi, K.; Kalay, H.; van't Hof, W.; Amerongen, A.V.; Veerman, E.C. Histatins are the major wound-closure stimulating factors in human saliva as identified in a cell culture assay. FASEB J. 2008, 22, 3805-3812.
[49] Steinstraesser, L.; Hirsch, T.; Schulte, M.; Kueckelhaus, M.; Jacobsen, F.; Mersch, E.A.; Stricker, I.; Afacan, N.; Jenssen, H.; Hancock, R.E.; et al. Innate defense regulator peptide 1018 in wound healing and wound infection. PLoS ONE 2012, 7, e39373.
[50] Kim, D.J.; Lee, Y.W.; Park, M.K.; Shin, J.R.; Lim, K.J.; Cho, J.H.; Kim, S.C. Efficacy of the designer antimicrobial peptide shapl in wound healing and wound infection. Amino Acids 2014, 46, 2333-2343.
[51] Di Grazia, A.; Luca, V.; Segev-Zarko, L.A.; Shai, Y.; Mangoni, M.L. Temporins a and b stimulate migration of hacat keratinocytes and kill intracellular Staphylococcus aureus. Antimicrob. Agents Chemother. 2014, 58, 2520-2527.
[52] van Kilsdonk, J. W. J.; Jansen, P. A. M.; van den Bogaard, E. H.; Bos, C.; Bergers, M.; Zeeuwen, P. L. J. M.; Schalkwijk, J. The Effects of Human Beta-Defensins on Skin Cells in vitro. Dermatology 2017,233, 155-163.
[53] Lai, Y.; Gallo, R.L. AMPed up immunity: How antimicrobial peptides have multiple roles in immune defense. Trends Immunol. 2009, 30, 131-141.
[54] Pickart, L.; Vasquez-Soltero, J. M.; Margolina, A. GHK Peptide as a Natural Modulator of Multiple Cellular Pathways in Skin Regeneration. Biomed Res Int. 2015, 2015:648108.
[55] Choudary, D.; Insen, S.G.; Goyal, S.; Chabbra, U.; Singal, G. A comparative study of collagen dressings versus conventional dressings in wound healing in chronic ulcers. J. Evol. Med. Dent. Sci. 2017, 6, 361-363.
[56] Ahmed, S.; Ikram, S. Chitosan based scaffolds and their applications in wound healing. Achiev. Life Sci. 2016, 10, 27-37.
[57] Kwon, Y.W.; Heo, S.C.; Lee, T.W.; Park, G.T.; Yoon, J.W.; Jang, I.H.; Kim, S.C.; Ko, H.C.; Ryu, Y.; Kang, H.; et al. N-acetylated proline-glycine-proline accelerates cutaneous wound healing and neovascularization by human endothelial progenitor cells. Sci. Rep. 2017, 7, 43057.
[58] Demidova-Rice, T.N.; Geevarghese, A.; Herman, I.M. Bioactive peptides derived from vascular endothelial cell extracellular matrices promote microvascular morphogenesis and wound healing in vitro. Wound RepairRegen. 2011, 19, 59-70.
[59] Banerjee, P.; Suguna, L.; Shanthi, C.Wound healing activity of a collagen-derived cryptic peptide. Amino Acids 2015, 47, 317-328.
[60] Tang, J.; Liu, H.; Gao, C.; Mu, L.; Yang, S.; Rong, M.; Zhang, Z.; Liu, J.; Ding, Q.; Lai, R. A small peptide with potential ability to promote wound healing. PLoS ONE 2014, 9, e92082.
[61] Mu, L.; Tang, J.; Liu, H.; Shen, C.; Rong, M.; Zhang, Z.; Lai, R. A potential wound-healing-promoting peptide from salamander skin. FASEB J. 2014, 28, 3919-3929.
[62] Fife, C.; Mader, J.T.; Stone, J.; Brill, L.; Satterfield, K.; Norfleet, A.; Zwernemann, A.; Ryaby, J.T.; Carney, D.H. Thrombin peptide chrysalin® stimulates healing of diabetic foot ulcers in a placebo-controlled phase i/ii study. Wound Repair Regen. 2007, 15, 23-34.
[63] Sheets, A.R.; Demidova-Rice, T.N.; Shi, L.; Ronfard, V.; Grover, K.V.; Herman, I.M. Identification and characterization of novel matrix-derived bioactive peptides: A role for collagenase from santyl(r) ointment in post-debridement wound healing? PLoS ONE 2016, 11, e0159598.
[64] Demidova-Rice, T.N.;Wolf, L.; Deckenback, J.; Hamblin, M.R.; Herman, I.M. Human platelet-rich plasma and extracellular matrix-derived peptides promote impaired cutaneous wound healing in vivo. PLoS ONE 2012, 7, e32146.
[65] Kwon, Y.W.; Heo, S.C.; Jang, I.H.; Jeong, G.O.; Yoon, J.W.; Mun, J.H.; Kim, J.H. Stimulation of cutaneous wound healing by an fpr2-specific peptide agonist wkymvm. Wound Repair Regen. 2015, 23, 575-582.
[66] Xiao, Y.; Reis, L.A.; Feric, N.; Knee, E.J.; Gu, J.; Cao, S.; Laschinger, C.; Londono, C.; Antolovich, J.; McGuigan, A.P.; et al. Diabetic wound regeneration using peptide-modified hydrogels to target re-epithelialization. Proc. Natl. Acad. Sci. USA 2016, 113, E5792-E5801.
[67] Yoo, Y. H.; Kim, Y. R.; Kim, M. S.' Lee, K.-J.; Park, K. H.; Hahn, J.-H. YAC tripeptide of epidermal growth factor promotes the proliferation of HaCaT keratinocytes through activation of EGFR. BMB Reports, 2014, 47(10), 581-586.
[68] Vigor ,C.; Rolfe, K. J.; Richardson, J.; Baker, R.; Grobbelaar, A.; Linge, C. The involvement of the ECM and RGD peptides in apoptosis induction during wound healing. J PlastReconstrAesthet Surg. 2006, 59:S4-S4.
[69] Namjoshi ,S.; Caccetta, R.; Benson, H. A. Skin peptides: biological activity and therapeutic opportunities J Pharm Sci. 2008, 97(7): 2524-42.

## Claims

1. The subject of the invention is a new compound with the general formula:
**xₙ[A-/-B]yₙ**
where:
Xₙ - represents a functional group/protecting group at the *N*-terminus of the compound, for example, for n=1H-, for n=2 Ac-, for n=3 -PEG2-
Yₙ- represents a functional group/protecting group at the C-terminus of the compound, for example, for n=1 - NH₂, for n=2 - COOH
A - represents a moiety containing the sequence**R¹K²I³E⁴I⁵V⁶R⁷K⁸K⁹P¹⁰I¹¹F¹²,** or its fragments B - represents a moiety containing the sequence **R¹L²I³D⁴R⁵T⁶N⁷A⁸N⁹F¹⁰L¹¹** , or its fragments -/- means a variation of the order and ways of combining A and B, A with A, B with A in any order and multiplicity; the methods of combining the motifs include: of the polyethylene glycol (PEG) motif, one or more polypeptides and/or one or more side chains of amino acids of any length and sequence, as well as motif A and/or B in a cyclic form.

2. The subject of the invention is a method for the preparation of a new compound as defined above, prepared in the following steps:
a) swelling Tenta Gel R RAM amide resin;
b) deprotecting the Fmoc protecting group with 20% piperidine in DMF;
c) washing the resin with DMF (4x);
d) performing a chloranil test;
e) performing a coupling reaction of the Fmoc-protected amino acid assisted by microwave irradiation and using a 0.5 M solution of DIC in DMF and 1M Oxyma Pure;
f) washing the resin with DMF (4x);
g) performing a chloranil test;
h) repeating steps b) to g) until the peptide of claim 1 is obtained;
i) cleaving the peptide from the support, while removing the protecting groups from the side-chains, with the mixture TFA:TIPSI:H₂O (92:4:4, v/v/v);
j) draining, precipitating with cooled ether, washing the precipitate three times with Et₂O, dissolving the precipitate and freeze-drying;
k) analysing and purifying with HPLC;
l) confirming the product's identity by mass spectrometry.

3. A pharmaceutical composition **characterized in that** it comprises a new compound as defined in claim 1 and at least one pharmaceutically acceptable carrier and/or diluent.

4. A pharmaceutical composition according to claim 3, **characterized in that** it can be applied locally or systemically, such as intravenously, orally, parenterally and/or in the form of implants, as well as by rectal application.

5. A pharmaceutical composition according to claims 3 - 4, **characterized in that** the local or system applications can be in solid and/or liquid form.

6. The composition according to claims 3-6, wherein the stimulation of wound healing is applicable.

7. The in vitro use of a new compound as defined in claim 1 for detecting stimulation of wound healing caused by mechanical, chemical, thermal, radiation, surgical or pathological defects.

8. Use according to claim 7, wherein the stimulation of wound healing occurs through accelerated epidermal wound healing.

9. Use according to claims 7-8, **characterized in that** wound healing involves wounds of the skin, epidermis, hair follicles, hair and sweat glands.

10. The novel compounds as defined in claim 1 for use as a means to promote skin wound healing, epidermis, hair follicles, hair, sweat glands.
